(19) Europäisches Patentamt
European Patent Office
Office européen des brevets

(11) EP 3 581 185 A1

(12) EUROPEAN PATENT APPLICATION
published in accordance with Art. 153(4) EPC

(43) Date of publication:
18.12.2019 Bulletin 2019/51

(51) Int Cl.:
A61K 31/575 (2006.01)        A61K 31/718 (2006.01)
A61K 45/06 (2006.01)         A61K 9/00 (2006.01)
A61K 9/08 (2006.01)

(21) Application number: 18751361.9

(22) Date of filing: 09.02.2018

(86) International application number:
PCT/KR2018/001770

(87) International publication number:
WO 2018/147685 (16.08.2018 Gazette 2018/33)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(30) Priority: 09.02.2017 KR 20170018220
08.02.2018 KR 20180015944

(71) Applicant: Yoo's Biopharm Inc.
Seoul 08501 (KR)

(72) Inventors:
• SONG, Yeong Ho
  Seoul 08201 (KR)
• KO, Hwi Jin
  Seoul 08294 (KR)

(74) Representative: Viering, Jentschura & Partner
mbB
Patent- und Rechtsanwälte
Am Brauhaus 8
01099 Dresden (DE)

(54) **COMPOSITION CONTAINING URSODEOXYCHOLIC ACID FOR PREVENTION OR TREATMENT OF VISUAL IMPAIRMENT**

(57) This disclosure relates to a composition for the prevention or the treatment of visual impairments comprising ursodeoxycholic acid (UDCA). More particularly, this disclosure relates to an excellent pharmaceutical composition for the prevention or the treatment of visual impairments such as macular degeneration, glaucoma and diabetic retinopathy which can be formulated for an oral administration, an intravitreal injection, or an eye drop administration by aqueous solubilized ursodeoxycholic acid.

FIG. 7C
YSB201-1

EP 3 581 185 A1

**Description**

**Field**

This disclosure relates to a composition for the prevention or the treatment of visual impairments comprising ursodeoxycholic acid (UDCA). More particularly, this disclosure relates to a pharmaceutical composition being capable for an oral administration, an intravitreal injection, intravenous injection, or an eye drop administration by aqueous solubilized ursodeoxycholic acid, and excellent for the prevention or the treatment of visual impairments such as macular degeneration, glaucoma, and diabetic retinopathy.

**Description of Related Art**

Macular degeneration, glaucoma and diabetic retinopathy are the three most common eye diseases that are the leading causes of blindness. The major causes of blindness are age-related eye diseases associated with an aging society. According to a study conducted in the United Kingdom in 2006, these three most common blindness diseases account for about 74% of all blindness diseases.

About 2% of adults age 40 and older have glaucoma. Glaucoma is a group of eye diseases that damages the optic nerve of the eye and finally causes vision loss by an abnormal increase in internal eye pressure. Glaucoma can be divided into 'primary glaucoma', which is related to cardiovascular diseases or diabetes, and 'secondary glaucoma', which is caused by cataract, uveitis, or eye surgery complications. There are no early warning signs or symptoms, but the vision of people with glaucoma can only be narrower and darker than that of the normal one. Glaucoma is also easily left untreated because there are no symptoms at the beginning, and if the optic nerve is damaged, there is no way to restore it with medication or surgery. Prevention and early detection are the solutions to slow the progression of the disease.

Diabetic retinopathy is a complication that occurs in circulatory disturbances of the peripheral blood vessels of the retina. Diabetic retinopathy affects most of those who have diabetes for 15 years or more. Diabetic retinopathy is classified as non-proliferative diabetic retinopathy and proliferative diabetic retinopathy. The non-proliferative diabetic retinopathy, which accounts for approximately 80% of diabetic retinopathy, is a disease in which breakdown of retinal capillaries result in fluid leaking into the center of the retina and causing difficulties with color discrimination and difficulties with night vision. Proliferative diabetic retinopathy is caused by the growth of abnormal new blood vessels due to lack of oxygen in the blood vessels, which easily burst due to the effect of diabetes and can cause large hemorrhages in the eye (vitreous hemorrhage), leading to retinal detachment and eventually serious visual impairment. In addition, fibrous tissues next to the new blood vessels proliferate and later contract to cause the retina supposed to be flattened to wrinkle and re-bleed, which further leads to complete loss of vision. Even though laser treatment (retinal photocoagulation) and surgical treatment (vitrectomy) are currently available, the successful recovery of visual acuity is often unsatisfactory since diabetic retinopathy causes overall damage to the retina.

Macular degeneration is the most common cause (57.2%) of blindness and visual impairment among the three most common eye diseases (British Journal of Ophthalmology, 2006), and is a dangerous disease that can cause vision loss due to the degenerative changes of the macula at the central portion of the retina with aging. Macular degeneration typically occurs in elderly people, wherein the risk of getting macular degeneration increases for those over the age of 65 and the prevalence is nearly 30% for those over the age of 75. In Korea, the number of patients with macular degeneration is increased to 140,000 in 2013 (40% increase from 2009 to 2013, published by National Health Insurance Review & Assessment Service) and the prevalence has been rapidly increased to 6.4% over 40 years old and 16.5% over 65 years old (2008 to 2012, published by Korea National Health and Nutrition Examination Survey in 2012) due to changes in the environment such as aging and using the computer.

Macular degeneration or age-related macular degeneration is clinically divided into two types: "dry (atrophic and non-exudative)" and "wet (neovascular and exudative)". Of these, severe loss of (eye) vision is usually seen in wet macular degeneration, but 20% of blindness due to this disease is also caused by dry macular degeneration. Dry or non-neovascular macular degeneration is a condition in which lesions such as atrophy of the retinal pigment epithelium or drusen are formed in the retina. The photoreceptor cells of the macula slowly break down and the visual acuity gradually decreases over time, and it is likely to progress toward wet macular degeneration. Wet or neovascular macular degeneration may cause choroidal neovascularization under the retina, resulting in severe vision distortion due to hemorrhage and exudation from the new blood vessels. It may cause blindness in a certain period of time later after onset.

Factors that may increase these macular degenerative diseases include smoking, hypertension, cholesterol, obesity, atherosclerosis, family history, etc. in addition to aging. They may also be caused by the side effects of the treatments for other diseases including malaria treatment such as chloroquine. However, the precise cause is unknown yet, and apoptosis of the cells related to the macula is considered to be the main cause.

In the case of dry macular degeneration, there is no fundamental treatment. Therefore, it is only satisfied with symptomatic treatment such as taking an antioxidant, but cannot prevent the progression of dry macular degeneration or treat the disease. In addition, there is no cure method for inhibiting apoptosis of macular-related cells even in the case of wet macular degeneration. The only methods to prevent and treat visual impairment are intravitreal injection of an antibody therapeutic agent for inhibiting the activity of vascular endothelial growth factor (VEGF), focal laser treatment or photodynamic therapy depending on the type and the position of neovascularization alone or a combination therapy with an intravitreal injection of an antibody therapeutic agent.

Treatment methods of macular degeneration approved by the FDA include Visudyne drug treatment, intravitreal injection with anti-vascular endothelial growth factor antagonist such as Lucentis® or Eylea®, Macugen®, implantable telescope and the like.

Visudyne is the very first drug therapy for the treatment of wet macular degeneration. However, according to the clinical trial results, the group treated with Visudyne has statistically significant visual acuity compared to those who had placebo treatment at the end of the one-year treatment period (Visudyne 86%, placebo 67%), but no significant difference at the end of the two-year treatment period (Visudyne 79%, placebo 72%).

Macugen® is a substance that inhibits the activity of vascular endothelial growth factors and is administered once every six weeks by intravitreal injection into the eyeballs. In clinical trials, 33% of the group treated with Macugen has showed sustained or improved visual acuity compared to 22% of the control group. Macugen® actually lowers the rate of visual impairment in patients with macular degeneration caused by aging. However, Macugen® has lower rate of preventing progression to vision loss and of improving vision than Lucentis® or Eylea®. In addition, less than 1% of the Macugen-treated patients have serious side effects such as endophthalmitis or retinal detachment, and 40% of the patients complain of side effects such as vitreous floaters or discomfort in the eyes.

The antibody drug of Lucentis® has been approved by the FDA in June, 2006 and is more effective than Visudyne® or Macugen® in treating wet (age-related) macular degeneration. Lucentis® has the ability to inhibit the activity of vascular endothelial growth factor (VEGF) and is administered once every four weeks by intravitreal injection into the eye. Lucentis® exhibits 40% vision improvement rate and 90% vision maintenance effect. However, intravitreal injection generally gives patients a sense of fear and discomfort.

Another antibody drug of Eylea® has been approved by the FDA in November, 2011 and is also a therapeutic agent with the ability to inhibit the activity of vascular endothelial growth factor in wet (age-related) macular degeneration like Lucentis®. Eylea® is administered once every 8 weeks after 3 initial monthly doses by intravitreal injection into the eyeballs. Eylea® has the same therapeutic effect as Lucentis®, but with fewer intravitreal injections. Eylea® is also administered by intravitreal injection, which causes fear and discomfort to patients during injection like Lucentis®.

Implantable telescope has been approved by the FDA in July, 2010 and is assistive device that expands the image on the retina to improve the damaged central vision in patients with severe macular degeneration. However, it does not prevent the progression of macular degeneration or improve macular degeneration.

The macular degeneration treatment methods together with their respective disadvantages and side effects described above result in considerable treatment costs for the patient and the corresponding country. The Ministry of Health and Welfare of Korea has revised 「Regulations on the standards and methods for medical care expenses」to expand the application of national health insurance benefits including increase of the number of times of use of macular degeneration agents and replacement treatment. Insurance coverage of macular degeneration agents (Lucentis®, Eylea®) has increased from 10 times to 14 times since November 1, 2014. When the macular degeneration agent is provided 14 times to each of 140,000 patients, the health insurance subsidies cost about KRW 14 million per patient and about KRW 2 trillion for the total number of patients.

In order to overcome the disadvantages of the conventional anti-vascular endothelial growth factor antagonists which are expensive and provide fear and discomfort of intravitreal injection, there have been many efforts for the development of low-molecular synthetic compounds including bile acids such as ursodeoxycholic acid (UDCA) or tauroursodeoxycholic acid (tUDCA), which can be metabolized into UDCA *in vivo,* for the treatment of wet (exudative or age-related) macular degeneration.

Some scientists around the world have demonstrated that UDCA or tUDCA can protect and treat retinal cells in retinal degenerative animal models. However, the key is formulation and delivery system for delivering a drug into the body, which is whether it has industrial availability. Ultimately, an important factor is how to easily deliver a therapeutically active amount into the eyeballs of the human body without side effects while increasing patient's convenience.

Intravitreal injection method can be invented by dissolving UDCA/tUDCA in water. However, there is no successful example of intravitreal injection of UDCA due to its practically insoluble chemical property in water up to date. The crystalline type of ursodeoxycholic acid has the acid dissociation constant, pKa, of 5.1, has an acicular structure, and is practically insoluble in water. Thus, it does not dissolve uniformly in water at a desired concentration. If UDCA is not aqueous solubilized uniformly in water, it cannot be absorbed well when it is administered to the inside of eyeball, cannot be washed off in tears, and can cause irritation due to residual crystalline forms of UDCA. Therefore, there has been no UDCA preparation for intravitreal injection, which is safe in the eye and does not cause inflammation and abnormal reaction of the retina after intravitreal injection.

Subcutaneous injection method of bile acid has also been tried. Dr. Boatright of Emory University, USA, conducted an experiment to test whether treatment of mice undergoing retinal degeneration could be inhibited with tauroursodeoxycholic acid (tUDCA) and found that tUDCA greatly slowed retinal degeneration in mice (Non-patent document 1). However, the drug delivery method was to dissolve tUDCA in a sodium carbonate buffer and subcutaneously inject it at the nape of the neck near the eyeball. The reason for using tUDCA, which is a water soluble metabolite of UDCA, instead of UDCA, is that its solubility in water is slightly higher than that of UDCA (solubility of UDCA; 20mg/t; solubility of tUDCA; 200mg/t). However, the crystalline form of tUDCA is practically insoluble in water. It is thus first dissolved in DMSO solution and then mixed with a PBS (pH 7.2) solution in a ratio of 1:4 to have solubility up to about 200 mg/t. Despite this, the stability of the solution can be maintained for only about 1 day. In addition, since the crystalline form of tUDCA is a strong hydrophilic taurine metabolite of UDCA, it is difficult to penetrate across the cell membranes of the human body and also difficult to make any preparation form due to its strong acidity (pH: 1 or less). That is, even when tUDCA is solubilized in a sodium carbonate buffer, tUDCA precipitates out after a certain period of time, so that it is difficult to maintain the stability of the drug formulation for more than one day. Thus, it is not suitable for the use as a drug due to poor stability. Although it is dissolved in water at a low concentration, there is no safety data on the absorption, distribution, metabolism and excretion, which shows that it is not precipitated out in the human body and thus safe after subcutaneous injection. It is also dangerous to provide subcutaneous injection at the nape of the neck as done in mice since there is no known subcutaneous injection at the nape of the neck for eye or intraocular treatment in the human body. Furthermore, tUDCA is more expensive than UDCA due to its high manufacturing cost and its toxicity, side effects, and/or mechanism of action are still unknown. Thus, there are many problems in commercializing tUDCA to be used for intravitreal injection.

Intraperitoneal injection of bile acid has also been tried. Dr. Sejoon Woo of the Seoul National University College of Medicine conducted an experiment to test whether UDCA and tUDCA could inhibit the development of choroidal neovascularization when it was dissolved in a sodium carbonate buffer and intraperitoneally injected to mice (Non-patent document 2). However, intraperitoneal injection cannot be applied to the human body since intraperitoneal injection is rarely used in the human body as a drug delivery system.

Alternatively, oral administration of the crystalline form of UDCA (tablets, capsules) could be tried to deliver it to the blood vessel and the eyeballs. However, up to now, there has been no example or attempt reporting oral administration of the crystalline form of UDCA to deliver a therapeutically active amount of UDCA to the inside of eyeballs through blood-retinal barriers (BRB) for the treatment of macular degeneration.

The reason is that, first of all, ursodeoxycholic acid has planar amphipathic molecule characteristics having both a hydrophobic surface without any substituents and a hydrophilic surface with hydroxyl groups and it exists in protonated form like other dihydroxy-bile acids. Thus, it is substantially insoluble in water (solubility: $53\mu M$). UDCA is also classified as an entero-hepatic circulation material. When taken orally, it is slightly soluble in the vicinity of the duodenum, absorbed by 95% or more to the liver through the small intestine, and again reaches the small intestine. Because of the high hepatic first-pass clearance, all the amount of the absorbed drug, taken through oral administration, is removed by perfusion through the portal vein, so that the amount entering the systemic circulation through the blood is very small. In other words, there is little chance of mass transfer to blood. Therefore, it is necessary to devise specific formulations or delivery systems in order to deliver a large amount of crystalline form of UDCA to the blood by oral administration. In addition, the retinal blood vessel has a blood-retinal barrier (BRB) structure having selective permeability to protect retinal nerve cells from external substances. In other words, the BRB structure is an optional barrier to prevent toxic substances flowing along with the blood from entering the retina and destroying the nerve tissues and to protect the nerve cells. The BRB structure is composed of both an inner (inner BRB) and an outer barrier (outer BRB). The inner BRB is formed of tight junctions with retinal capillary endothelial cells to protect the retinal nerve from external toxic substances, and retinal pigment epithelial cell of outer BRB functions to selectively block the movement of substances from the leaky choroid to the sub-retinal space to protect the retina. Any preparation for oral administration of UDCA, which can easily deliver the bile acid into the eyeballs across the blood-retinal barrier as well as within the blood, has not yet been developed.

Furthermore, an eye drop preparation can be invented. However, the crystalline form of UDCA is classified as a skin irritant and has the acid dissociation constant, pKa, of about 5.0, which is acidic in water. Thus, there is a serious disadvantage in development as an eye drop preparation because it can cause skin irritation when it is in contact with the eye or around the eye. That is, since this crystalline form of UDCA has an acicular structure with a very sharp structure, when it comes into contact with the eye, it may enter into the cornea, surrounding tissues, pores and wounds. Since the pH of the eye is 7.4, the crystalline form of UDCA does not dissolve in this condition and does not wash down, but keeps staying on the spot while constantly irritating the contact area to cause inflammation. Therefore, direct use as an eye drop is not appropriate unless there is any specific formulation or delivery system for the eye drop.

Furthermore, intravenous injection can be considered. However, as described for the eye drop, because UDCA is practically insoluble in water and precipitates out at a pH of around 7.0 and thus cannot be solubilized uniformly in water in the form of a single molecule, it may block blood vessels or inflammation when administered in the blood vessels so that direct use as intravenous injection is not appropriate unless there is any specific formulation or delivery system for the intravenous injection.

There have been no commercially available preparations for delivering the bile acid which can easily deliver a therapeutically active amount of the crystalline form of UDCA into the eyeballs without side effects, be applicable to the human body, provide comfort to the patient and increase therapeutic effects. Thus, any preparation for delivering the bile acid, which can be just orally administered to deliver a therapeutically active amount of UDCA/tUDCA across the blood-retinal barrier to the eyeballs without adverse effects and provides equal therapeutic efficacy which the existing antibody drug, Lucentis® or Eylea®, does, can be the best formulation in terms of improving patient convenience and improving visual acuity by eliminating various disadvantages associated with the intravitreal injection.

In light of the foregoing research results, it is necessary to develop appropriate preparations to eliminate irritation caused by chemical properties of the bile acid having a strong acidity and an acicular structure and be well soluble in water in a high concentration without causing precipitation in order to efficiently deliver UDCA or tUDCA, which is effective in preventing the retina degradation, to the retina, and appropriate delivery systems to the eyeballs such as an oral administration, an intravitreal injection, or an eye drop administration. Among these, the most preferred pharmaceutical preparation is an oral administration which can be used non-invasively so as not to cause fear and pain to the patient. That is, the oral administration alone allows the therapeutically active amount of UDCA/tUDCA to reach the blood and further to the retina within the eyeballs across the blood-retina barrier to protect the retina. Until now, there has been no attempt to deliver UDCA/tUDCA to the inside of the eyeballs across the blood-retinal barrier by oral administration or intravitreal injection in order to protect the retina and to prevent or treat visual impairments.

## SUMMARY

An object of this disclosure is to resolve problems associated with the crystalline form of UDCA which is classified as a skin irritant, is strongly acidic, has acicular crystal structure, causes irritation when directly contacted with the eye due to insolubility in water, causes adverse reactions to the retina upon intravitreal injection, and is harmful to human body. That is, an object of this disclosure is to provide a composition for the prevention or the treatment of visual impairments such as macular degeneration, glaucoma and diabetic retinopathy, wherein ursodeoxycholic acid is aqueous solubilized in water to be a clear aqueous solution form to be used as intravitreal injection or eye drop.

Another object of this disclosure is to resolve problems associated with the crystalline form of UDCA which is self-associated in the form of micelles even though it is hardly aqueous solubilized in water due to its unique chemical properties of UDCA having both hydrophilic and hydrophobic properties at the same time. That is, another object of this disclosure is to provide a composition for the prevention or the treatment of visual impairments such as macular degeneration, glaucoma and diabetic retinopathy, wherein UDCA is aqueous solubilized in water to be a clear aqueous solution form not to form precipitates and cause self-association at a pH of about 7.4, which is the tear pH, or in any pH in the human body without adverse reactions to the retina.

Still another object of this disclosure is to resolve problems associated with the crystalline form of UDCA which is classified as an enterohepatic circulating material due to its chemical properties when taken orally and cannot be delivered to the blood and further to the eyeballs at a high concentration due to the high hepatic first-pass clearance. That is, another object of this disclosure is to provide a composition for the prevention or the treatment of visual impairments such as macular degeneration, glaucoma and diabetic retinopathy, wherein UDCA is aqueous solubilized in water to be a clear aqueous solution form to deliver a therapeutically active amount into the eyeballs across the blood plasma and blood-retinal barrier by an oral administration alone.

It is therefore an object of this disclosure to provide a composition for the prevention or the treatment of visual impairments such as macular degeneration, glaucoma and diabetic retinopathy comprising aqueous solubilized ursodeoxycholic acid, which increases the intraocular absorption with a therapeutically active amount by oral administration alone in order to eliminate pain and fear of intravitreal injection and to increase patient's convenience.

Still another object of this disclosure is to provide a composition for the prevention or the treatment of visual impairments, wherein UDCA is aqueous solubilized in water to be a clear aqueous solution form to effectively deliver UDCA/tUDCA to the eyeballs without causing inflammation or side effects by intravitreal injection or oral administration alone not only to inhibit the development of choroidal neovascularization but also to promote recovery of retinal function and prevent the expression of vascular endothelial growth factor(VEGF).

Still another object of this disclosure is to provide a composition for the prevention or the treatment of visual impairments having excellent drug stability, which can prevent self-association of the crystalline form of ursodeoxycholic acid molecules caused by unique chemical properties of the ursodeoxycholic acid having both hydrophilic and hydrophobic properties at the same time, be mixed well with hydrophilic and hydrophobic materials when eye drop and intravitreal injection are prepared, and does not form precipitates even after a long period of time.

Still another object of this disclosure is to provide a composition for the prevention or the treatment of macular degeneration diseases, which can provide a synergistic effect to prevent or treat macular degeneration diseases by co-administration with a conventional therapeutic agent for the age-related macular degeneration disease such as a protein antagonist including Lucentis® and Eylea® by intravitreal injection or co-administration with Visudyne.

According to one aspect of this disclosure, there is provided a composition for the prevention or the treatment of visual impairments comprising active ingredients of: (a) ursodeoxycholic acid (UDCA); (b) an aqueous soluble starch conversion product; and (c) water, wherein the composition comprises a clear aqueous solution of an aqueous solubilized ursodeoxycholic acid for all pH values.

According to one embodiment of this disclosure, the composition may be prepared for an intravitreal injection, delivering the UDCA to the retina without causing skin irritation or inflammation in the eye after intravitreal injection.

According to one embodiment of this disclosure, a single dose of the UDCA of the composition for the intravitreal injection may be 50-100 μl at a concentration of 0.1-1.5 mg/ml.

According to one embodiment of this disclosure, the composition may be prepared for an oral administration, delivering the UDCA to the blood and then further delivering a therapeutically active amount thereof to the eyeball across the blood-retinal barrier.

According to one embodiment of this disclosure, a daily dose of the UDCA of the composition for the oral administration may be 5-30 mg/kg.

According to one embodiment of this disclosure, the composition may be orally administered at least once a day for 20 days or more.

According to one embodiment of this disclosure, the UDCA of the composition may start to be distributed in the eye within 5-10 minutes after oral administration and stay for a certain time, about 1 hour, and then wash-out.

According to one embodiment of this disclosure, the composition may be prepared for an intravenous injection to be administered directly to the blood without blocking blood vessels and causing skin irritation.

According to one embodiment of this disclosure, the composition may be administered as an eye drop.

According to one embodiment of this disclosure, the UDCA of composition administered as an eye drop may be carried from outside the eye to inside without causing skin irritation or adverse reactions around the eye or the eyeball.

According to one embodiment of this disclosure, a single dose of the UDCA of the composition for the eye drop may be 30 - 50 μl at a concentration of 0.1 - 2.0 mg/ml.

According to one embodiment of this disclosure, the number of eye drops of the UDCA of the composition may be 1-10 times a day, but it is not limited thereto.

According to one embodiment of this disclosure, wherein the visual impairment may be selected from macular degeneration, glaucoma, and diabetic retinopathy.

According to one embodiment of this disclosure, the visual impairment may be macular degeneration.

According to one embodiment of this disclosure, the visual impairment may be wet age-related macular degeneration.

According to one embodiment of this disclosure, the composition may have at least one of functions of inhibiting the development of choroidal neovascularization, promoting the recovery of retinal function, and regulating the expression level of vascular endothelial growth factor (VEGF).

According to one embodiment of this disclosure, the UDCA may be an aqueous solubilized ursodeoxycholic acid selected from an aqueous soluble ursodeoxycholic acid, an aqueous soluble ursodeoxycholic acid derivative, an ursodeoxycholic acid salt, and a ursodeoxycholic acid conjugated with an amine.

According to one embodiment of this disclosure, the UDCA may be at least one aqueous solubilized ursodeoxycholic acid selected from an ursodeoxycholic acid (UDCA), a tauroursodeoxycholic acid (tUDCA) and a glycoursodeoxycholic acid (gUDCA).

According to one embodiment of this disclosure, the UDCA may be present in a therapeutically active amount.

According to one embodiment of this disclosure, the UDCA may be used in an amount of 0.01 - 5 parts by weight based on the total weight of the composition.

According to one embodiment of this disclosure, the UDCA may be used in an amount of 0.04 - 0.16 parts by weight based on the total weight of the composition.

According to one embodiment of this disclosure, the aqueous soluble starch conversion product may be maltodextrin and the maltodextrin may be used in an amount of 1 - 70 parts by weight based on the total weight of the composition.

According to one embodiment of this disclosure, the pH value of the composition may be 3 - 9, and the aqueous soluble starch conversion product may be maltodextrin, and the minimum weight ratio of the ursodeoxycholic acid to the maltodextrin may be 1:16 - 1:30.

According to one embodiment of this disclosure, the pH value of the composition may be 6.5 - 8, and the aqueous soluble starch conversion product may be maltodextrin, and the minimum weight ratio of the ursodeoxycholic acid to the maltodextrin may be 1:13 - 1:30.

According to one embodiment of this disclosure, the aqueous soluble starch conversion product may be at least one selected from maltodextrin, dextrin, liquid glucose, corn syrup solid, soluble starch, dextran, guar gum, pectin and soluble non-starch polysaccharide.

According to one embodiment of this disclosure, the composition may be formulated into a syrup form, a cream form, a paste form or a dried form.

According to one embodiment of this disclosure, the composition may be administered together with a therapeutic agent for macular degeneration.

According to one embodiment of this disclosure, the therapeutic agent for macular degeneration may be an anti-vascular endothelial growth factor antagonist.

According to one embodiment of this disclosure, the therapeutic agent for macular degeneration may be prepared for an intravitreal injection.

The composition for the prevention or the treatment of visual impairments according to an embodiment of this disclosure is a form of ursodeoxycholic acid preparation in which ursodeoxycholic acid is aqueous solubilized in water as a clear aqueous solution so as to resolve fundamental problems associated with conventional crystalline form of UDCA causing skin irritation when directly contacted with the eye.

In the composition for the prevention or the treatment of visual impairments according to an embodiment of this disclosure, the UDCA is aqueous solubilized in water at a high concentration in a single molecule form and stays stable for a certain period of time. Thus, it can be formulated for intravitreal injection which was impossible with the conventional crystalline form of UDCA due to self-association thereof.

The composition for the prevention or the treatment of visual impairments according to an embodiment of this disclosure is a form of ursodeoxycholic acid preparation in which ursodeoxycholic acid is aqueous solubilized in water as a clear aqueous solution at a high concentration so as to resolve problems associated with conventional crystalline form of UDCA preparation (tablet, capsule), which cannot carry UDCA at a high concentration in the blood as being classified as an entero-hepatic circulation material. Thus, it can deliver a therapeutically active amount of UDCA to the eyeballs through the blood vessel and across the blood-retinal barrier when administered orally. Therefore, a sufficient amount of UDCA effective for the treatment can be delivered to the eyeballs by oral administration alone, thereby eliminating the pain and fear of intravitreal injection to patients and improving patient's convenience.

The composition for the prevention or the treatment of visual impairments according to an embodiment of this disclosure is well absorbed without causing any abnormal reaction to the retina during intravitreal injection not only to inhibit the development of choroidal neovascularization but also to promote recovery of retinal function and regulated the expression level of vascular endothelial growth factor (VEGF).

The composition for the prevention or the treatment of visual impairments according to an embodiment of this disclosure can be used as an eye drop by solving irritation of the eye, which is the disadvantage of conventional crystalline form of UDCA. Therefore, it is possible to eliminate pain and fear of intravitreal injection to patients and to increase patient's convenience.

The composition for the prevention or the treatment of visual impairments according to an embodiment of this disclosure allows providing eye drop and intravitreal injection preparations for the prevention or the treatment of visual impairments with high stability which does not cause discomfort such as harmful foreign materials to and irritation in the eye and does not precipitate with pH changes.

According to one embodiment of this disclosure, it is possible to provide a composition for the prevention or the treatment of macular degeneration diseases that can efficiently transfer tUDCA or gUDCA, which is an *in vivo* metabolite of UDCA, or UDCA, into the eyeballs without any abnormal reaction of the retina.

According to one embodiment of this disclosure, the composition for the prevention or the treatment of macular degeneration diseases can achieve equal or better preventive or therapeutic effects of macular degeneration diseases to or than Lucentis® or Eylea®, which is an intravitreal injectional protein antagonist.

According to another embodiment of this disclosure, the composition for the prevention or the treatment of macular degeneration diseases can be synergistically effective for treating macular degeneration diseases by administering it together with a known macular degeneration therapeutic agent.

According to one embodiment of this disclosure, the composition for the prevention or the treatment of macular degeneration diseases can achieve an effect of preventing or treating macular degeneration diseases by oral administration or eye drops in addition to intravitreal injection. This method of oral administration or eye drops has the advantage of maximizing the convenience to patients for the treatment of macular degeneration.

According to one embodiment of this disclosure, the composition for the prevention or the treatment of macular degeneration diseases is a low-molecular-weight chemical compound, so that it can be manufactured with low cost compared to high cost antibody drugs.

Accordingly, this disclosure can effectively prevent or treat visual impairment diseases such as macular degeneration, glaucoma, and diabetic retinopathy at a lower cost than an antibody drug such as Lucentis® or Eylea®.

Other objects and features of this disclosure will become more apparent from the following detailed description, the drawings, and the claims.

**BRIEF DESCRIPTION OF THE DRAWINGS**

FIG. 1 illustrates whether a clear aqueous solution is formed or not based on pH value of the ursodeoxycholic acid solution prepared in Example 3 of this disclosure.

FIG. 2 illustrates whether a clear aqueous solution is formed or not based on pH value of the ursodeoxycholic acid solution prepared in Example 4 of this disclosure.

FIG. 3 illustrates whether a clear aqueous solution is formed or not based on pH value of the ursodeoxycholic acid solution prepared in Example 5 of this disclosure.

FIG. 4 illustrates whether a clear aqueous solution is formed or not based on pH value of the ursodeoxycholic acid solution prepared in Example 6 of this disclosure.

FIG. 5 illustrates whether a clear aqueous solution is formed or not based on pH value of the ursodeoxycholic acid solution prepared in Example 7 of this disclosure.

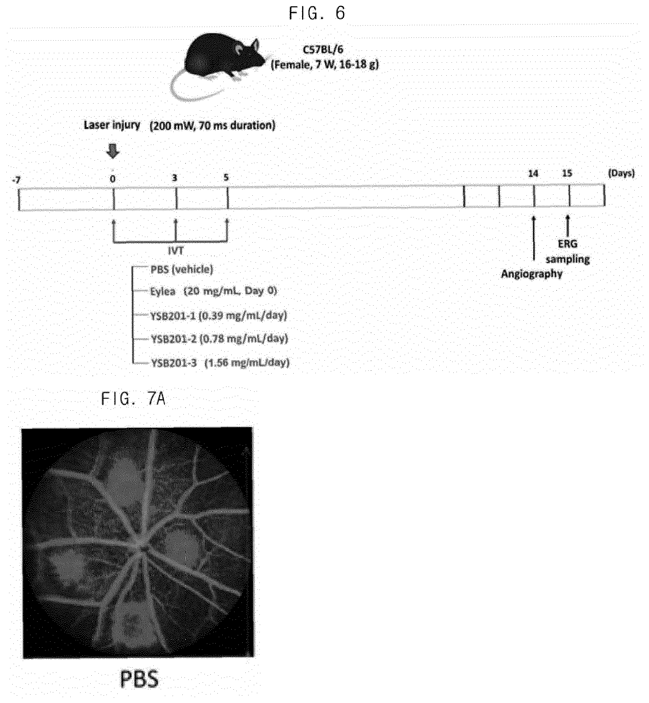

FIG. 6 is a mimetic diagram illustrating an overall test method of evaluating the efficacy in a laser-induced choroidal neovascularization (CNV) animal model in order to determine an anti-angiogenic activity by the intravitreal injection of the composition of this disclosure, wherein the mouse is treated with laser and then injected with 2 $\mu$L of PBS (phosphate buffered saline) or Eylea® (10 mg/mL) as control, or 2 $\mu$L of aqueous solubilized ursodeoxycholic acid of YSB201-1 (0.39 mg/mL, Example 8), YSB201-2 (0.78 mg/mL, Example 9) or YSB201-3 (1.56 mg/mL, Example 10) to each of two eyeballs three times at 2-day intervals.

FIG. 7A - FIG. 7F are fluorescence images and a graph of quantified fluorescence illustrating comparative reduction in laser-induced choroidal damage (neovascularization) after intravitreal injection using YSB201-1 (Example 8), YSB201-2 (Example 9), or YSB201-3 (Example 10) according to the embodiments of this disclosure or a conventional VEGF antibody drug, Eylea® (control group).

FIG. 8A - FIG. 8F are optical coherence tomography images of retina and a graph of quantified CNV lesions illustrating comparative reduction in laser-induced choroidal damage (neovascularization) after intravitreal injection using YSB201-1 (Example 8), YSB201-2 (Example 9), or YSB201-3 (Example 10) according to the embodiments of this disclosure or a conventional VEGF antibody drug, Eylea® (control group).

FIG. 9A - FIG. 9G are graphs illustrating electroretinogram (ERG) measured at day 15 using a single white light under the dark-adapted state after the mouse with laser-damaged retina was treated three times with YSB201-1 (Example 8), YSB201-2 (Example 9), or YSB201-3 (Example 10) according to the embodiments of this disclosure or once with Eylea® (control group) through intravitreal injection.

FIG. 10 is a diagram illustrating the effect on the expression level of vascular endothelial growth factor (VEGF) in the choroid and the retina damaged by laser after the mouse with laser-damaged retina was treated with YSB201-1 (Example 8), YSB201-2 (Example 9), or YSB201-3 (Example 10) according to the embodiments of this disclosure or Eylea® (control group) through intravitreal injection.

FIG. 11 is a mimetic diagram illustrating an overall test method of evaluating the efficacy in a laser-induced choroidal neovascularization (CNV) animal model in order to determine an anti-angiogenic activity by the oral administration of the composition of this disclosure, wherein the mouse was orally administered with olive oil (control group) or an aqueous solubilized ursodeoxycholic acid of YSB201-4 (125 mg/kg/day, Example 11) or YSB201-5 (250 mg/kg/day, Example 12), once a day from 10 days before laser injury to the retina of the mouse to 10 days after laser injury.

FIG. 12A - FIG. 12D are fluorescence images and a graph of quantified fluorescence illustrating comparative reduction in laser-induced choroidal damage (neovascularization) after oral administration of YSB201-4 (Example 11) or YSB201-5 (Example 12) according to embodiments of this disclosure.

FIG. 13A - 13D are optical coherence tomography images of retina and a graph of quantified CNV lesions illustrating comparative reduction in laser-induced choroidal damage (neovascularization) after oral administration of YSB201-4 (Example 11) or YSB201-5 (Example 12) according to embodiments of this disclosure.

FIG. 14A - FIG. 14E are graphs illustrating electroretinogram (ERG) measured at day 15 using a single white light under the dark-adapted state after the mouse was orally administered with olive oil (control group) or an aqueous solubilized ursodeoxycholic acid, YSB201-4 (Example 11) or YSB201-5(Example 12) once a day from 10 days before laser injury to the retina of the mouse to 10 days after laser injury.

FIG. 15 is an image measuring the effect on the expression level of vascular endothelial growth factor (VEGF) in the choroid and the retina damaged by laser after the mouse with laser-damaged retina was orally administered with YSB201-4 (Example 11) or YSB201-5 (Example 12) according to the embodiments of this disclosure.

FIG. 16 and FIG. 17 are pharmacokinetic (PK) data illustrating the changes in the concentration of bile acids delivered into plasma over time after oral administration of YSB201.

FIG. 18A, FIG. 18B, and FIG. 19 are pharmacokinetic (PK) data illustrating the changes in the concentration of bile acids delivered into the eyeballs over time after oral administration of YSB201. In FIG. 18B, UDCA type bile acids indicate the sum of concentrations of UDCA and tUDCA and gUDCA, which are *in vivo* metabolites thereof, having cytoprotective effect, and other bile acids that function as a surfactant indicate the sum of concentrations of other bile acids other than those three kinds of bile acids.

FIG. 20 and FIG. 21 are pharmacokinetic (PK) data illustrating the changes in the concentration of bile acids delivered into stomach over time after oral administration of YSB201.

FIG. 22 is pharmacokinetic (PK) data illustrating the changes in the sum of the concentrations of UDCA and tUDCA and gUDCA, which are *in vivo* metabolites thereof, and the sum of concentrations of other bile acids delivered to plasma over time after oral administration of YSB201.

FIG. 23 is pharmacokinetic (PK) data illustrating the changes in the sum of the concentrations of UDCA and tUDCA and gUDCA, which are *in vivo* metabolites thereof, and the sum of concentrations of other bile acids delivered to the eyeballs over time after oral administration of YSB201.

FIG. 24 is pharmacokinetic (PK) data illustrating the changes in the sum of the concentrations of UDCA and tUDCA and gUDCA, which are *in vivo* metabolites thereof, and the sum of concentrations of other bile acids delivered to stomach over time after oral administration of YSB201.

## DETAILED DESCRIPTION

In order that the invention may be more readily understood, certain terms are first defined herein for convenience. Unless otherwise defined herein, the scientific and technical terms used in this disclosure shall have the meaning generally understood by those who are skilled in the art. Unless clearly used otherwise, expressions in the singular number include a plural meaning, and those in the plural number include a singular meaning.

As used herein, the term "treating" or "treatment" encompasses preventing, ameliorating, mitigating and/or managing visual impairments and/or conditions by the administration of a composition of this disclosure.

As used herein, the term "comprising as an active ingredient" or "comprising a therapeutically active amount" is meant to include a certain amount of an active ingredient, enough to provide the effects for the prevention and the treatment of visual impairments as a composition, a composition for intravitreal injection, a composition for oral administration, a composition for eye drop, and a composition for intravenous injection.

As used herein, the term "prevention" means all actions that at least reduce parameters, for example the degree of symptoms, associated with the conditions to be treated after a drug is orally administered from before a visual impairment is occurred to after.

The terms "clear aqueous solution" or "clear aqueous solution" used in this disclosure mean a transparent aqueous solution in a solution state in which there are substantially no visually observed precipitates in naked eye.

The term "visual impairment" used in this disclosure includes macular degeneration, glaucoma and diabetic retinopathy.

According to one aspect of this disclosure, there is provided a composition for the prevention or the treatment of visual impairments including: (a) ursodeoxycholic acid(UDCA); (b) an aqueous soluble starch conversion product; and (c) water, wherein the composition comprises a clear aqueous solution of an aqueous solubilized ursodeoxycholic acid for all pH values.

The ursodeoxycholic acid is hydrophilic bile acid, which can be administered orally. The ursodeoxycholic acid in the human body is as low as about 3% of total bile content, but it is also present in the bile of the human body. UDCA is the only US FDA-approved drug as a therapeutic agent for primary biliary cirrhosis.

The UDCA has pharmacological actions of antioxidant, anti-inflammatory, and anti-apoptosis. These actions are very important mechanisms in the treatment of visual impairments and are more pronounced when UDCA acts as a single molecule. Therefore, the key factor is to make the substance with such effects be absorbed and be delivered to the inside of the human eyeballs. The crystalline form of UDCA is classified as an irritant and is an amphipathic molecule that has both hydrophilic and lipophilic properties, so it is almost insoluble in water. Even when aqueous solubilized in a small amount, it is in the form of dimers, tetramers, or micelles. It is thus difficult to function as a UDCA single molecule. However, the composition of this disclosure enables the crystalline form of UDCA to be solubilized at a high concentration in water to function as a single molecule.

The aqueous solubilized ursodeoxycholic acid is stabilized with maltodextrin in water and as a result, the solubility of pure ursodeoxycholic acid molecules in water can be increased by 3,000 times or more. The aqueous solubilized ursodeoxycholic acid, which is dissolved in water by the above method, exist as a single molecule form and nonionic molecular state having amphipathic properties due to its molecular nature, so that an absorption rate of the ursodeoxycholic acid can be drastically increased because it is absorbed *in vivo* by passive mechanism in addition to high intercellular and intracellular diffusion through fast dispersion by the concentration difference. All the take together, the aqueous solubilized ursodeoxycholic acid, in which an active ingredient of ursodeoxycholic acid is dissolved in water at a high concentration up to 60 g/L, is the most ideal multi-functional drug that can prevent, alleviate or treat visual impairments such as macular degeneration when it is applied through intravitreal injection, oral administration, intravenous injection or eye drop.

The composition of this disclosure may include, but is not limited to, the solubility of UDCA in the composition can be about 3,000 times higher than the conventional commercialized UDCA preparation (0.15 M vs. 0.05 mM) and can be about 300 times or much higher compared to the taurine-conjugated form of ursodeoxycholic acid (TUDCA) (0.15 M vs. 0.45 mM). However, it is not limited thereto. Accordingly, the applicant has completed this disclosure by using aqueous solubilized UDCA.

According to one embodiment of this disclosure, the composition may be prepared for intravitreal injection to deliver UDCA of the composition to the retina, and may not cause skin irritation or inflammation in the eye after injection.

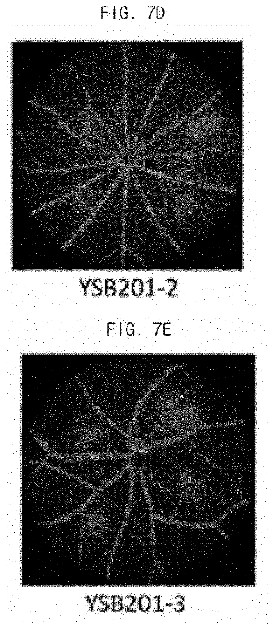

According to one embodiment of this disclosure, the composition for the intravitreal injection of this disclosure significantly inhibits the choroidal neovascularization of the retina, promotes regeneration of retinal cells, and down-regulates the expression level of vascular endothelial growth factor. According to experimental results of Examples related to the intravitreal injections of this disclosure and FIG. 7A - FIG. 10, it is clearly confirmed that when the composition is injected into the eyeballs of mice, the effect is equal to or better than that with the VEGF antibody injection agent, Eylea®, which is currently used for the treatment for macular degeneration.

According to one embodiment of this disclosure, a single dose of the UDCA of the composition for the intravitreal injection may be 50 - 100 $\mu$l at a concentration of 0.1-1.5 mg/ml, including but not limited to at a concentration of 0.1 - 3.0 mg/ml, preferably at a concentration of 0.1 - 1.5 mg/ml. When the UDCA concentration of the single injection is 0.1 or more, the effect is more obvious. When the UDCA concentration is more than 1.5 mg/ml, the effect is substantially equal to the effect shown with 1.5 mg/ml, so that an economically efficient amount can be provided. In the case of the eyeballs of the human body, the amount to be injected once a day may be 50 - 100 $\mu$l. However, it is not limited thereto.

According to one embodiment of this disclosure, the composition may be prepared for oral administration to deliver the UDCA to the blood and further deliver a therapeutically active amount into the eyeballs across the blood-retinal barrier.

When UDCA is administered orally in a tablet or capsule form which is the conventional formulation of the crystalline form of UDCA, about 30 - 60% of it is absorbed along the jejunum and ileum by nonionic passive diffusion and the crystalline structure of UDCA (crystalline form of UDCA), due to insolubility, is absorbed only by a small amount (up to 20% of intakes) at the ileum of the colon by the active transport mechanism. When UDCA is absorbed by hepatocytes, it can be conjugated to tUDCA and gUDCA which are excreted by hepatic first-pass clearance as bile acids secreted from the human body. Therefore, the concentration of UDCA in the blood after oral administration is very low. Accordingly, there has been no example provided with composition for the prevention or the treatment of visual impairments such as macular degeneration which delivers UDCA to the eyeballs.

Alternatively, according to one embodiment of this disclosure, the pharmacokinetic study results of the composition upon oral administration (125 mg/Kg, Example 11) to mice show that, unlike conventional crystalline form of UDCA formulations (tablets, capsules), the highest UDCA concentration in the blood is 36.56 $\pm$ 3.30 $\mu$g/mL, which is 12.7-fold higher, and $T_{max}$ is 5 minutes which is 48 times faster. Therefore, the high UDCA concentration in the blood can be achieved even with the same oral dose compared to the existing formulation and the preventive or therapeutic effect of visual impairments such as macular degeneration can be achieved.

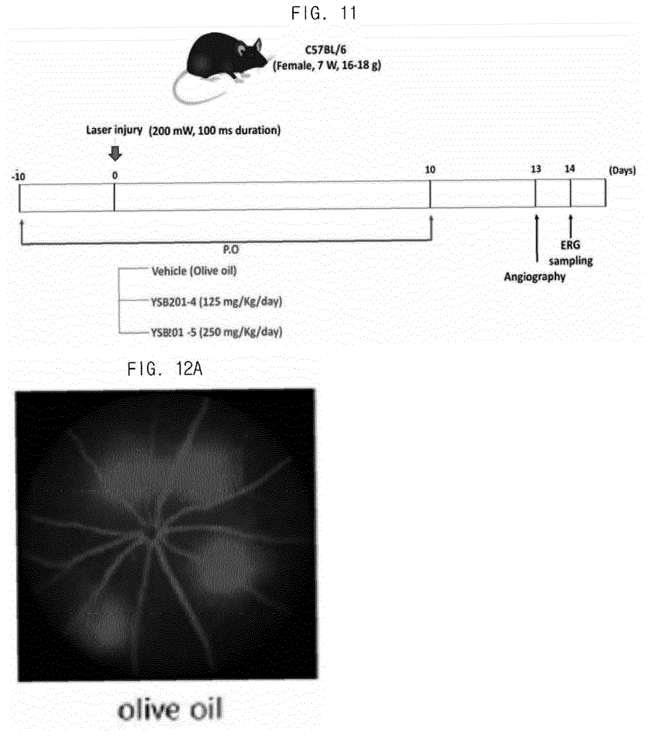

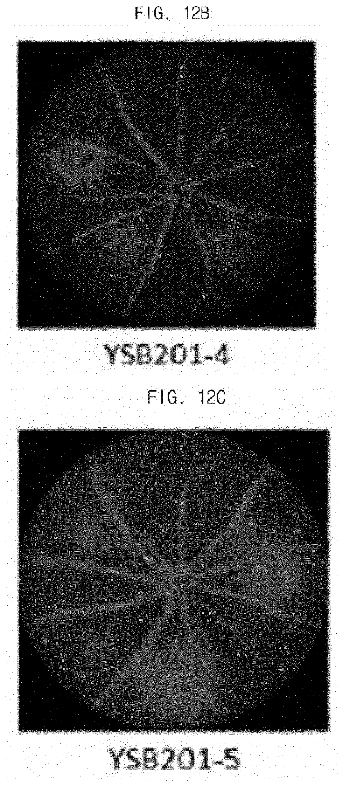

According to one embodiment of this disclosure, the composition for oral administration of this disclosure can remarkably inhibit the choroidal neovascularization of the retina, promote the regeneration of retinal cells, and down-regulate the expression level of vascular endothelial growth factor. According to experimental results of Examples related to the oral administration and FIG. 12A - FIG. 15, it is clearly confirmed that the composition is remarkably more effective when administered orally, compared with the control group.

According to one embodiment of this disclosure, a daily oral dose of UDCA of the composition may be 5 - 30 mg/kg. The effect is more obvious when the daily oral dose of UDCA is 5 mg/kg or more. The effect is substantially equivalent to that shown with 30 mg/kg when it exceeds 30 mg/kg, so that an economically efficient amount can be provided. The daily dose of UDCA of the composition may be 10 - 30 mg/kg, 15 - 30 mg/kg, 20 - 30 mg/kg, 25 - 30 mg/kg, 5 - 25 mg/kg, 10 - 25 mg/kg, 15 - 25 mg/kg, 20 - 25 mg/kg, 5 - 20 mg/kg, 10 - 20 mg/kg, 15 - 20 mg/kg, 5 - 15 mg/kg, and 10 - 15 mg/kg. However, it is not limited thereto. An interval of oral administration of UDCA may be 1 day, 2 days, 3 days, 4 days, 5 days, 6 days, and 7 days, depending on the symptoms. However, it is not limited thereto. In addition, the dose is suitable for the human body, but is not limited thereto.

According to one embodiment of this disclosure, the composition may be orally administered at least once a day for 20 days or more. However, it is not limited thereto.

According to one embodiment of this disclosure, the UDCA of the composition starts to be distributed in the eyeballs within 5 - 10 minutes after oral administration, and may stay in the eyeballs for a predetermined time, about 1 hour, and then wash out.

According to one embodiment of this disclosure, the composition may be administered directly to the blood through intravenous injection without blocking the blood vessel and causing skin irritation.

According to one embodiment of this disclosure, the composition may be administered as eye drop.

According to one embodiment of this disclosure, the UDCA of the composition administered as the eye drop can be delivered from the outside of the eye to the inside of the eye without causing skin irritation and adverse reactions around the eyes or the eye.

According to one embodiment of this disclosure, a single eye drop dose of UDCA of the composition may be 30 - 50 $\mu$l at a concentration of 0.1 mg/ml - 2.0 mg/ml, preferably a concentration of 0.1 mg/ml - 1.5 mg/ml. However, it is not limited thereto. When the single eye drop concentration of UDCA of the composition is more than 0.1 mg/ml, the effect is more obvious. When it is more than 2.0 mg/ml, the effect is substantially equivalent to that shown with 2.0 mg/ml, so that an economically efficient amount can be provided.

According to an embodiment of this disclosure, the appropriate number of drops per day of the UDCA of the composition may be one to ten times a day. However, it is not limited thereto.

The eye drop according to an embodiment of this disclosure may include a chelating agent. The chelating agent is not particularly limited as long as it is a compound chelating metal ions. Example of the chelating agent may include edetic acid (ethylenediamine tetra acetic acid) such as monosodium edetate, disodium edetate, trisodium edetate, tetrasodium edetate, dipotassium edetate, tripotassium edetate, tetrapotassium edetate and the like, or salts thereof; citric acid such as monosodium citrate, disodium citrate, trisodium citrate, monopotassium citrate, dipotassium citrate, tripotassium citrate and the like, or salts thereof; metaphosphoric acid such as sodium metaphosphate, potassium metaphosphate and the like, or salts thereof; pyrophosphoric acid such as sodium pyrophosphate, potassium pyrophosphate and the like, or salts thereof; polyphosphoric acid such as sodium polyphosphate, and potassium polyphosphate and the like, or salts thereof; malic acid such as monosodium malate, disodium malate, monopotassium malate, dipotassium malate and the like, or salts thereof; tartaric acid such as sodium tartrate, potassium tartrate and potassium sodium tartrate and the like, or salts thereof; phytic acid such as sodium phytate, potassium phytate and the like, or salts thereof. However, it is not limited thereto.

In addition, edetic acid, citric acid, metaphosphoric acid, pyrophosphoric acid, polyphosphoric acid, malic acid, tartaric acid, phytic acid, and salts thereof may include hydrates and organic solvates thereof.

In this disclosure, preferred examples of the chelating agent include edetic acid, a salt of edetic acid (edetate), citric acid, a salt of citric acid (citrate), metaphosphoric acid, a salt of metaphosphoric acid (metaphosphate), pyrophosphoric acid, a salt of (polyphosphate), and more preferred examples include sodium edetate (including a hydrate thereof such as disodium edetate hydrate and the like), citric acid (including a hydrate thereof such as citrate monohydrate and the like), sodium metaphosphate, and sodium polyphosphate.

The eye drop according to an embodiment of this disclosure may further include a preservative. Examples of the preservative may include benzalkonium chloride, benzethonium chloride, chlorhexidine gluconate, paraben, sorbic acid, chlorobutanol, boric acid, chlorite and the like, preferably benzalkonium chloride. However, it is not limited thereto.

The eye drop may further include a pharmaceutically acceptable additive, if necessary. Examples including a buffering agent such as sodium phosphate, sodium hydrogenphosphate, sodium dihydrogenphosphate, sodium acetate, epsilon-aminocaproic acid; an isotonizing agent such as sodium chloride, potassium chloride and concentrated glycerin; and a surfactant such as polyoxyethylene sorbitan monolete, polyoxyl 40 stearate and polyoxyethylene hardened castor oil may be selected and added as needed.

According to one embodiment of this disclosure, examples of the visual impairment may include macular degeneration, glaucoma, and diabetic retinopathy.

According to one embodiment of this disclosure, the visual impairment may be macular degeneration.

According to one embodiment of this disclosure, the visual impairment may be wet age-related macular degeneration.

According to one embodiment of this disclosure, the composition may function as one or more of the functions of inhibiting the development of choroidal neovascularization, promoting the recovery of retinal function, and regulating the expression level of vascular endothelial growth factor (VEGF).

According to one embodiment of this disclosure, the UDCA which is selected from an aqueous soluble ursodeoxycholic acid, an aqueous soluble ursodeoxycholic acid derivative, an ursodeoxycholic acid salt, and an ursodeoxycholic acid conjugated with an amine can be aqueous solubilized ursodeoxycholic acid. An aqueous soluble metal salt of ursodeoxycholic acid and an aqueous soluble O-sulfonated bile acid are also included as an aqueous soluble ursodeoxycholic acid salt. However, it is not limited thereto.

According to one embodiment of this disclosure, the UDCA may be at least one aqueous solubilized UDCA selected from a ursodeoxycholic acid (UDCA), a tauroursodeoxycholic acid (tUDCA) and a glycoursodeoxycholic acid (gUDCA). The tauroursodeoxycholic acid (tUDCA) and glycoursodeoxycholic acid (gUDCA) are *in vivo* metabolites or derivatives of UDCA. The tUDCA is a UDCA derivative which is the taurine conjugate form of UDCA and the gUDCA is a UDCA derivative which is the glycine conjugate form of UDCA.

According to one embodiment of this disclosure, the UDCA may be present in a therapeutically active amount. The therapeutically active amount means an amount enough to provide the effects for the prevention and the treatment of visual impairments as a composition, a composition for intravitreal injection, a composition for oral administration, a composition for eye drop, and a composition for intravenous injection such as an amount capable of preventing or treating a visual impairment.

According to one embodiment of the disclosure, the ursodeoxycholic acid is included in an amount of 0.01 - 5 parts by weight based on the total weight of the composition. If the amount of ursodeoxycholic acid is less than 0.01 part by weight based on the total weight of the composition, the effects for the prevention or the treatment of visual impairments may be insignificant. On the other hand, if the amount of ursodeoxycholic acid is more than 5 parts by weight, a clear aqueous solution may not be formed. However, it is not limited thereto. When cloudy precipitates are formed instead of a clear aqueous solution, it may be difficult to use it as an intravitreal injection, an oral administration agent, and an eye drop.

When precipitates are formed, ursodeoxycholic acid may not be dissolved in water and thus exist in a crystalline form of UDCA. When this is used for preparing eye drop or intravitreal injection, it may cause skin irritation due to the crystalline form of UDCA. Thus, the preparation of a clear aqueous solution is required to remove all of the crystalline form of UDCA that can cause skin irritation.

UDCA may be included in an amount of 0.01 - 5 parts by weight, including but not limited to 0.1 - 5 parts by weight, 1 - 5 parts by weight, 2 - 5 parts by weight, 3 - 5 parts by weight, 4 - 5 parts by weight, 0.01 - 3 parts by weight, 0.1 - 3 parts by weight, 1 - 3 parts by weight, 2 - 3 parts by weight, 0.01 - 2.5 parts by weight, 0.1 - 2.5 parts by weight, 1 - 2.5 parts by weight based on the total weight of the composition. For the intravitreal injection, UDCA may be included in an amount of 0.05 - 0.2 parts by weight, more preferably 0.04 - 0.16 parts by weight, and even more preferably 0.04 - 0.07 parts by weight based on the total weight of the composition. For the oral administration, UDCA may be included in an amount of 0.1 - 2.5 parts by weight, preferably 1 - 2.5 parts by weight based on the total weight of the composition.

According to one embodiment of this disclosure, the aqueous soluble starch conversion product is maltodextrin, and the maltodextrin is used in an amount of 1.0 - 70 parts by weight based on the total weight of the composition. However, it is not limited thereto. When the amount of maltodextrin is less than 1.0 part by weight, an effective amount of UDCA cannot be dissolved in water, resulting in poor effects for the prevention or the treatment of visual impairments. On the other hand, when the amount of maltodextrin is more than 70 parts by weight, precipitates are formed, resulting in skin irritation in the eye since UDCA or maltodextrin precipitates out of the aqueous solution.

The maltodextrin may be included in an amount of 1 - 60 parts by weight, including but not limited to 5 - 60 parts by weight, 10 - 60 parts by weight, 20 - 60 parts by weight, 30 - 60 parts by weight , 40 - 60 parts by weight, 50 - 60 parts by weight, 1 - 50 parts by weight, 5 - 50 parts by weight, 10 - 50 parts by weight, 20 - 50 parts by weight, 30 - 50 parts by weight, 40 - 50 parts by weight, 1 - 40 parts by weight, 5 - 40 parts by weight, 10 - 40 parts by weight, 20 - 40 parts by weight, 30 - 40 parts by weight, 1 - 30 parts by weight, 5 - 40 parts by weight, 10 - 30 parts by weight, 20 - 30 parts by weight, 1 - 20 parts by weight, 5 - 20 parts by weight, 10 - 20 parts by weight, 1 - 10 parts by weight, 5 - 10 parts by weight based on the total weight of the composition.

According to one embodiment of this disclosure, the aqueous soluble starch conversion product is maltodextrin, and the minimum weight ratio of maltodextrin to the ursodeoxycholic acid may be 1:30, including but not limited to 1:25, 1:20, 1:15, 1:12, or 1:6. An amount of the aqueous soluble starch conversion product with high molecular weight used in the composition can be defined as an aqueous solubilized amount of the selected ursodeoxycholic acid at a desired concentration and the pH range described herein. The minimum amount of maltodextrin may be equally applied to the case of tauroursodeoxycholic acid and glycoursodeoxycholic acid.

According to one embodiment of this disclosure, there is provided a composition for the prevention or the treatment of visual impairments, wherein the pH value is 3 - 9 and the aqueous soluble starch conversion product is maltodextrin, wherein minimum weight ratio of the UDCA to maltodextrin is 1:16 - 1:30. Minimum weight ratio of the UDCA to maltodextrin may be 1:16 - 1:20, 1:16 - 1:25, 1:16 - 1:30, 1:20 - 1:25, 1:20 - 1:30, or 1:25 - 1:30. However, it is not limited thereto.

When the pH value is from 3 or higher to less than 6 and the minimum weight ratio of UDCA to maltodextrin is 1:1 - 1:15, precipitates may be formed, resulting in no clear aqueous solution. However, it is not limited thereto.

According to one embodiment of this disclosure, there is provided a composition for the prevention or the treatment of visual impairments, wherein the pH value is 6 - 9, the aqueous soluble starch conversion product is maltodextrin, and the minimum weight ratio of UDCA to maltodextrin is 1:13 - 1:30. However, it is not limited thereto.

The aqueous soluble starch conversion product of this disclosure includes a carbohydrate obtained directly from partial or incomplete hydrolysis of starch under various pH conditions. Non-limiting examples of the aqueous soluble starch conversion product may include maltodextrin, dextrin, liquid glucose, corn syrup solid (dried powder of liquid glucose). The corn syrup solid may be Maltrin M200 and the maltodextrin may be Maltrin M700, both of which are manufactured by GPC™ (Grain Processing Corporation), located in Muscatin, Iowa, USA. However, it is not limited thereto.

If the starch conversion product consists of a polymer, the polymer may include at least one reducing end and at least one non-reducing end. The polymer may be linear or branched. The molecular weight may be about 100 mass units or more, or $10^6$ mass units or more. The high molecular weight aqueous soluble starch conversion product, though not limited thereto, may have a molecular weight of $10^5$ mass units or more.

According to one embodiment of this disclosure, the composition may further include a soluble non-starch polysaccharide. The soluble non-starch polysaccharide may be obtained under various pH conditions by various hydrolysis or synthesis mechanisms. Non-limiting examples of the soluble non-starch polysaccharide may include dextran, guar gum, pectin, indigestible soluble fibers, and the like. If the soluble non-starch polysaccharide consists of a polymer, the polymer may have at least one reducing end and at least one non-reducing end. The polymer may be a linear or branched polymer. The molecular weight of the polysaccharide of this disclosure may be at least about 100 mass units, or at least about $10^6$ mass units, preferably at least $10^5$ mass units. However, it is not limited thereto. The composition may be provided as a composition which is an aqueous solution comprising a combination of the aqueous soluble starch conversion product and/or the aqueous soluble non-starch polysaccharide.

According to one embodiment of this disclosure, the minimum weight ratio of ursodeoxycholic acid to liquid glucose (e.g., corn syrup) needed to prevent precipitation of the composition is about 1:25 (i.e., about 12.5 g per 500 mg of ursodeoxycholic acid in 100 ml of water or about 25 g per 1 g of ursodeoxycholic acid in 200 ml of water). However, it is not limited thereto.

In addition, the minimum amount of dried powder of liquid glucose (corn syrup solids, e.g., Maltrin M200) needed to prevent precipitation of the composition from the dosage form of this disclosure is about 30 g per 1 g of ursodeoxycholic acid in 100 ml of water, or about 60 g per 2 g of ursodeoxycholic acid in 200 ml of water. However, it is not limited thereto.

The minimum amount of the soluble non-starch polysaccharide required to prevent precipitation of the composition from the dosage form of this disclosure is about 50 g of guar gum per 500 mg of ursodeoxycholic acid in 100 ml of water, or 80g of pectin per 500 mg of ursodeoxycholic acid in 100 ml of water. However, the minimum amount of the soluble non-starch polysaccharide or aqueous soluble starch conversion product with high molecular weight may be determined mainly by the absolute amount of ursodeoxycholic acid in the solution preparation rather than the concentration.

The composition of this disclosure may further include dietary fiber when formulated for oral administration. Non-limiting examples of the dietary fiber include guar gum, pectin, psyllium, oat rubber, soybean fiber, oat bran, corn hull, cellulose, and wheat bran.

The composition of this disclosure may further include an emulsifying agent and a suspending agent. Non-limiting examples of the emulsifying agent may include guar gum, pectin, acacia, carrageenan, sodium carboxymethylcellulose, hydroxymethylcellulose, hydroxypropylcellulose, methylcellulose, polyvinylalcohol, povidone, tragacanth gum, xanthan gum and sorbitan ester.

The composition of this disclosure may further include a pharmaceutically acceptable additive. Non-limiting examples of the pharmaceutically acceptable additive may include starch, gelatinized starch, microcrystalline cellulose, lactose, povidone, colloidal silicon dioxide, calcium hydrogenphosphate, lactose, mannitol, glutinous, arabic gum, pregelatinized starch, corn starch, powder cellulose, hydroxypropylcellulose, opadry, sodium starch glycolate, carnauba wax, synthetic aluminum silicate, stearic acid, magnesium stearate, aluminum stearate, calcium stearate, white sugar, dextrose, sorbitol and talc. The pharmaceutically acceptable additive according to this disclosure is preferably included in the composition in an amount of 0.1 - 90 parts by weight. However, it is not limited thereto. In addition, the composition of this disclosure may be administered in various forms of parenteral administration at the time of actual clinical administration. In the case of formulation, a diluent or excipient such as a filler, an extender, a binder, a wetting agent, a disintegrating agent, and a surfactant may be added. Examples of parenteral administrations may include sterilized aqueous solutions, non-aqueous solutions, suspensions, emulsions, freeze-dried preparations, suppositories, and injections.

According to one embodiment of this disclosure, there is provided a composition for the prevention or the treatment of visual impairments, wherein the pH of the composition is in the range of 1 - 10, and wherein the composition is in a clear aqueous solution state at the pH value. The composition may be solubilized in water and may be in the form of a clear aqueous solution without precipitation at the pH described above. That is, the composition may be in the form of a clear aqueous solution without precipitation of UDCA even after several months (1, 2, 3, 4, 5, 6, 7, 8, 9, 10, 11, or 12 months). A selected pH range that does not precipitate the ursodeoxycholic acid and the aqueous soluble starch conversion product in the composition may be about pH 1 - about pH 10, preferably about pH 3 - about pH 9, more preferably pH 6 - pH 8, and most preferably pH 6.5 - pH 8. However, it is not limited thereto. The composition may further include an acid, a base and a buffering agent if it is necessary to maintain the pH described above. The pH adjusting material may be, but is not limited to, HCl, $H_3PO_4$, $H_2SO_4$, $HNO_3$, CH3COOH, citric acid, malic acid, tartaric acid, lactic acid, phosphate, eidetic acid, and alkali. The properties and methods for using such pH adjusting materials are well known in the art. The pH range is the pH level of any subset that can be obtained in an aqueous system sufficient to allow various formulations to remain in solution from the preparation and to be injected to the eyeballs or be administered orally to the blood, depending on the method of administration. Thus, the composition may be used as a formulation in solution, without the composition according to this disclosure being precipitated at the pH level of the mouth, stomach, and intestines.

According to some embodiments of this disclosure, ursodeoxycholic acid remains dissolved under acidic conditions as a free ursodeoxycholic acid even though it is generally insoluble under acidic conditions. The composition may further include another composition in which the composition remains soluble when added to any formulation. In addition, the composition may provide a clear and stable solution for providing the composition for the prevention or the treatment of visual impairments such as macular degeneration diseases in the form of intravitreal injection, oral administration, eye drop, or intravenous injection.

According to one embodiment of this disclosure, the composition may be formulated into a syrup form, a cream form, a paste form, or a dried form. The syrup may be, but not limited to, normal syrup or thick syrup.

According to one embodiment of this disclosure, the composition may be dried and formulated in powder form. The powder-type composition can be stored and handled easily and can be easily formulated into a desired form.

According to one embodiment of this disclosure, the composition may be administered in combination with another therapeutic agent for macular degeneration diseases.

According to one embodiment of this disclosure, another therapeutic agent for macular degeneration diseases may be an anti-vascular endothelial growth factor antibody. Examples thereof may include Eylea®, Avastin®, Macugen®, Lucentis® or a combination thereof. However, it is not limited thereto. In addition, the composition may be administered in combination with Visudyne injection. However, it is not limited thereto.

According to one embodiment of this disclosure, the therapeutic agent for the macular degeneration disease may be for intravitreal injection.

This disclosure contains extensive information on the current awareness of the genetics, biochemistry, and cell biology of macular degenerative diseases, but future research may reveal that aspects of these perceptions are either inaccurate or incomplete. Thus, those skilled in the art will understand that this disclosure is not limited to a particular model or mechanism of action whether part of this disclosure is taken or not.

In addition, those skilled in the art will recognize that other equivalent or alternative compositions and methods may be utilized. For example, although it has been described that a plurality of compounds can be administered together with aqueous solubilized ursodeoxycholic acid, it is understood that other compounds may also be included.

Also, the application of another drug may be performed at the same time as the administration of the aqueous solubilized ursodeoxycholic acid composition of this disclosure, or they may be administered separately in the same or overlapped time period (for example, at the same time, the same date, or the same week).

Hereinafter, this disclosure will be described in more detail with reference to Examples.

## Examples

### Materials and Methods

#### <Contract Research Organization>

1) Preparation for animal models of macular degeneration and animal test of pharmacokinetics - T2B Infrastructure Center for Ocular Disease of Inje University Busan Paik Hospital
2) Pharmacokinetic study - College of Pharmacy, Inje University

#### < Preparation for animal models of macular degeneration >

1) All animals were acclimatized for approximately 7 days to be adapted to the laboratory environment.
2) The animals that did not show any clinical sign of diseases or wounds and showed appropriate weight were used in the study. A control group and a treatment group of the animals were randomly assigned on the basis of the most recently measured body weight.
3) The mice were housed in the group of three in a mice cage during the entire test period.
4) The test environment was constantly controlled to have 19 - 20°C of temperature, 40 - 60% of humidity, and 150 - 300 Lux of room light.
5) After C57 BL/6 mice (Orient Bio, Korea) were acclimatized to be adapted to the environment for 1 week, 7-week old mice were treated with the image-guided laser (Phoenix, USA) especially optic nerve area at 3, 6, 9, and 12 o'clock positions. The laser was irradiated at 532 nm, 100 ms/70 ms, 200 mW, and a spot size was 50 $\mu$m.

< Analysis of Macular Degeneration Therapeutic Effects >

The following tests were performed to determine the effects of samples on inhibition of the development of choroidal neovascularization and recovery of retinal cell functions using the mouse model of laser-induced choroidal neovascularization (CNV).

1) Fundus Fluorescein Angiography (FFA)

Both eyes of CNV mice were dilated and intraperitoneally injected with 1% fluorescein (Sigma, USA) to dye the blood vessels. The mice were anesthetized and fluorescent fundus images of the laser-induced neovascularization were taken with the retinal imaging microscope (Micron IV image) at 5 minutes after fluorescein injection. The CNV lesions were represented by the corrected total fluorescence (CTF) calibrated by using Image J program. Here, CTF can be calculated by the following equation.

$$*CTF= \text{(Integrated Density)}-[\text{(Area of selected lesion)} \times \text{(Mean fluorescence of background readings)}]$$

2) Optical Coherence Tomography (OCT)

After both eyes of CNV mice were dilated and anesthetized, tomography of the laser-induced neovascularization was taken using the image-guided OCT system by positioning the pupils dilated mice in front of the OCT system and the guide line in the center of the CNV with guidance of bright-field live fundus image to scan each formed choroidal neovascularization.

3) Electroretinography (ERG)

The mice were dark adapted for 24 hours before test and all tests were performed in a darkroom. The eyes of mice were dilated and general anesthetized by intraperitoneal injection with a mixture of ketamine (30 mg/kg) and xylazine hydrochloride (2.5 mg/kg) in order to detect the function of the retina. Electrodes were placed in skin, tail and cornea to run electroretinography. A single white light was used to stimulate the retina to obtain electrical activity of the retina. The amplitude from the trough of A-wave to the peak of B-wave was measured and evaluated as an index of the function of the retina.

4) Western blot analysis

After 15 days from laser injury, the mice were euthanized and the choroid and retinal layers were separated by removing the sclera, cornea and lens from the eyeballs after extracting the eyeballs. The choroidal and retinal tissues were washed twice with PBS, and proteins were extracted by homogenizing the tissue with Pro-PREP (iNtRON, Korea). The extracted proteins were quantified using the BCA protein assay kit (Thermo scientific, USA) and 20 $\mu$g of the proteins were used for Western blotting. After blocking the membrane with 5% skimmed milk for 1 hour, the primary antibody was diluted to 1:1000 in TBS-T and the membrane was incubated in the primary antibody solution overnight at 4 °C. After washing the membrane with TBS-T, the secondary antibody was diluted to 1: 5000 in 3% skimmed milk, the membrane was incubated in the secondary antibody solution at room temperature for 1 hour, and then target proteins were detected using a Chemi image system.

< Analysis of pharmacokinetics of aqueous solubilized UDCA >

For the pharmacokinetic study, a test sample YSB201-4 was orally administered to C57BL/6 mouse model, and the pharmacokinetic trends of the test sample in the plasma and the living organs were analyzed over time.

1) Methods

(1) The test sample was orally administered to mice, and blood and various tissue samples were collected at each hour. An organic solvent was used to extract the drug components in the tissue samples, and the concentration thereof was quantitatively analyzed by HPLC fluorescence detector. The tissue samples were taken at 0, 5, 10, 30 minutes and 1, 2, 4, 10, 24, 48, and 72 hours after oral administration. Blood and tissue samples were collected from 4 mice at each hour. Blood and tissue samples from the control group having the fasting and dietary treatments as in the test group were collected at 0, 4, 10, 24, 48, and 72 hours.

(2) A suitable extraction method was established by extracting the test sample components in the tissue sample using an organic solvent and by calculating its recovery (%). The high-performance liquid chromatography (HPLC) was used in order to quantitatively analyze the bile acid components in various biological tissues, using enzyme reaction and fluorescence detection.

(3) Pharmacokinetic parameters after oral administration were calculated by applying the results of concentration analysis of drug components in blood and tissues to WinNonLin, which is the pharmacokinetic analysis program. The PK profile of the YSB201-4 preparation was confirmed.

(4) The animal laboratory of Indang Biomedical Research Center of Inje University Pusan Paik Hospital received a certificate from the Korea Ministry of Food and Drug Safety in 2014, and the test protocol was approved by the IACUC (Institutional Animal Care and Use Committee, IACUC No. IJUBPH_2016-001-02) of College of Medicine, Inje University.

2) Test samples and equipment

(1) YSB201-4 was used as a test sample.

(2) The various bile acids used as standard substances were gUDCA, tUDCA, UDCA, GCA (glycocholic acid hydrate), TCA (taurocholic acid sodium salt hydrate), CA (cholic acid), GCDCA (glycochenodeoxycholic acid), TCDCA (taurochenodeoxycholic acid), GDCA (glycodeoxycholic acid), TDCA (taurodeoxycholic acid), CDCA (chenodeoxycholic acid), DCA (deoxycholic acid), GLCA (glycolithocholic acid sodium salt), TLCA (taurolithocholic acid), and LCA (lithocholic acid). They were purchased from Sigma-Aldrich.

(3) The analytical equipment was the 2695 Alliance high performance liquid chromatography (HPLC) instrument from the US Water Company. BilePak II column (4.6125 mm, JASCO, Japan) and EnzymePak $3\alpha$-HSD column (4.635 mm, JASCO, Japan) were also used.

3) Drug administration method - oral administration

The animals were fasted for 12 hours immediately prior to oral administration, and then the dose of each individual was calculated based on the body weight measured after fasting for 12 hours prior to oral administration and orally administered using a disposable syringe equipped with sonde. Solid diet was re-fed after 4 hours from the oral administration.

4) Test animals

36 C57BL/6 mice (female) weighing 16 - 18 g were obtained from CoreTech Co., Ltd. (Pyongtaek, Gyeonggi-do, Korea) and acclimatized for 7 days and then used for the test. At the first administration, the mice with about $\pm$ 20% of the total average weight were used. The mice were housed at temperature of 19 - 25°C, humidity of 40 - 60%, and room light of 150 - 300Lux. Cleaning of the test room and the cage was performed according to the standard operation procedure of Indang Biomedical Research Center, Inje University Busan Paik hospital.

5) Experimental design for grouping and dosing

Experimental design for grouping and dosing for the control and test groups are shown in Table 1.

Table 1

| No | Group | Treatment | Oral dose mg/kg | Time after administration | No. of mice Female |
|---|---|---|---|---|---|
| 1 | Control | None | None | 4h | 3 |
| 2 | | | | 10h | 3 |
| 3 | | | | 24h | 3 |
| 4 | | | | 48h | 3 |
| 5 | | | | 72h | 3 |
| 6 | Test | YSB201 Oral admin istration (12.5mg/ml) | 125mg/kg | 0min | 3 |
| | | | | 5min | 4 |
| 7 | | | | 10min | 4 |
| 8 | | | | 30min | 4 |
| 9 | | | | 1h | 4 |
| 10 | | | | 2h | 4 |
| 11 | | | | 4h | 4 |
| 12 | | | | 10h | 4 |
| 13 | | | | 24h | 4 |
| 14 | | | | 48h | 4 |
| 15 | | | | 72h | 4 |

6) Sample analysis

An analysis system of bile acids of JASCO, Japan was used to analyze blood and biological samples of the mice. Concentrations of bile acids were quantitatively analyzed using a fluorescence detector (excitation: 345 nm, emission: 470 nm) of Waters® Alliance 2695 HPLC system. HPLC conditions are summarized in Table 2.

Table 2

| < HPLC Conditions > | |
|---|---|
| Items | Conditions |
| Separation Column | Guard column/BilePak II column (4.6 x 125 nm) |
| Enzyme Column | EnzymePak $3\alpha$-HSD column (4.6x35 mm) |
| Column Temp. | 25 °C (Column oven) |
| Sample Temp. | 10 °C (Auto Sampler) |
| Eluent | A: Acetonitrile/Methanol/30 mM ammonium acetate (30/30/40) B: Acetonitrile/Methanol/30 mM ammonium acetate (20/20/60) |
| Flow Rate | 1 mL/min |
| Reagent Solution | 0.3 mM NAD, 10 mM $KH_2PO_4$, 1 mM EDTA-2Na, 0.05% 2-mercaptoethanol, pH 7.8 (adjusting with KOH) |
| Reagent Solution Flow Rate | 1 mL/min |
| Fluorescence Wavelength | Excitation: 345 nm, Emission: 470 nm |

(continued)

| < HPLC Conditions > | | | | |
|---|---|---|---|---|
| Items | Conditions | | | |
| Eluent Gradient Condition | Time (min) | Eluent A (%) | Eluent B (%) | |
| | 0 | 0 | 100 | |
| | 32 | 100 | 0 | |
| | 60 | 100 | 0 | |
| | 65 | 0 | 100 | |
| Analysis Time | 65 min | | | |
| Injection Volume | 10 $\mu$L | | | |

7) Data and statistical analysis

Test results were summarized using Microsoft Excel 2010 and additional pharmacokinetic analyses were performed on the results of blood concentration analysis using the Pharsight WinNonlin 7.0 program (Certara, USA). The pharmacodynamic profile was evaluated using GraphPad Prism 5.0, wherein a P-value of 0.05 or less was considered significant.

## 1. Preparation of aqueous solubilized ursodeoxycholic acid in a clear aqueous solution

### (Example 1) A clear aqueous solution with 1:6 of weight ratio of UDCA to maltodextrin

A clear aqueous stock solution of aqueous solubilized UDCA containing natural UDCA and aqueous soluble starch having low dextrose equivalent was prepared.

Particularly, 6.7 g of sodium hydroxide pellets were aqueous solubilized in 400 ml of purified water. 60 g of UDCA was dissolved in the sodium hydroxide solution while stirring at room temperature. 360 g of maltodextrin was added slowly to the clear solution while stirring. A preservative was then added in an amount appropriate for the pharmaceutical formulation to the clear solution obtained by performing ultrasonication (750W, 20kHz) at high throughput and the pH was adjusted by the dropwise addition of HCl. Purified water was added and adjusted to be a total of 1,000 ml. If necessary, the clear solution was filtered through a suitable filtration apparatus. This filtration is important for removing impurities from the raw material or sterilization, but it is not intended to remove the granular material because the solution is already clear. As shown in Table 3, the prepared ursodeoxycholic acid solution formed a clear aqueous solution at pH 10.3, 9.2, and 6.7 without visual precipitation, but formed precipitates at pH 5.4.

### (Example 2) A clear aqueous solution with 1:12 of weight ratio of UDCA to maltodextrin

A clear aqueous stock solution of aqueous solubilized UDCA containing natural UDCA and aqueous soluble starch having low dextrose equivalent was prepared.

Particularly, it was prepared in accordance with the same procedure as in Example 1, except that 720 g of maltodextrin as one high molecular weight aqueous soluble starch conversion product per 60 g of ursodeoxycholic acid was used. As shown in Table 3, the prepared ursodeoxycholic acid solution formed a clear aqueous solution at pH 9.6, 7.3, 6.5, and 6.0 without any visible precipitation, but formed precipitates at pH 5.5.

### (Example 3) A clear aqueous solution with 1:15 of weight ratio of UDCA to maltodextrin

A clear aqueous stock solution of aqueous solubilized UDCA containing natural UDCA and aqueous soluble starch having low dextrose equivalent was prepared.

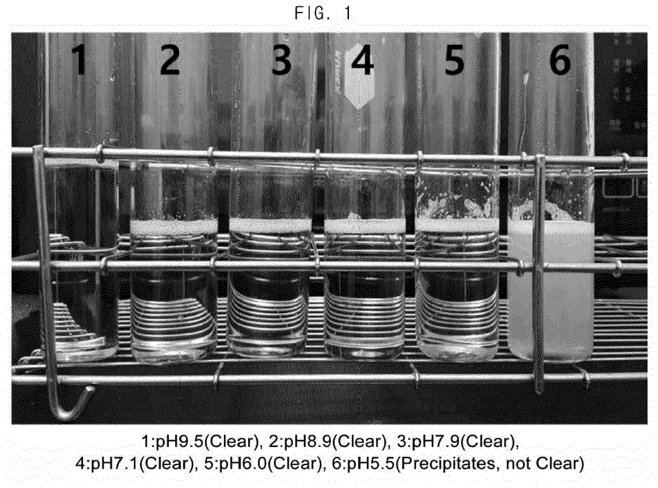

Particularly, it was prepared in accordance with the same procedure as in Example 1, except that 750 g of maltodextrin as one high molecular weight aqueous soluble starch conversion product per 50 g of ursodeoxycholic acid was used. 5.7 g of sodium hydroxide pellets were dissolved in 400 ml of purified water and then used. As shown in Table 3, the prepared ursodeoxycholic acid solution formed a clear aqueous solution at pH 9.5, 8.9, 7.9, 7.1, and 6.0 without visual precipitation, but formed precipitates at pH 5.5. FIG. 1 is images illustrating whether a clear aqueous solution of the ursodeoxycholic acid solution is formed or not at each pH value.

**(Example 4) A clear aqueous solution with 1:20 of weight ratio of UDCA to maltodextrin**

A clear aqueous stock solution of aqueous solubilized UDCA containing natural UDCA and aqueous soluble starch having low dextrose equivalent was prepared.

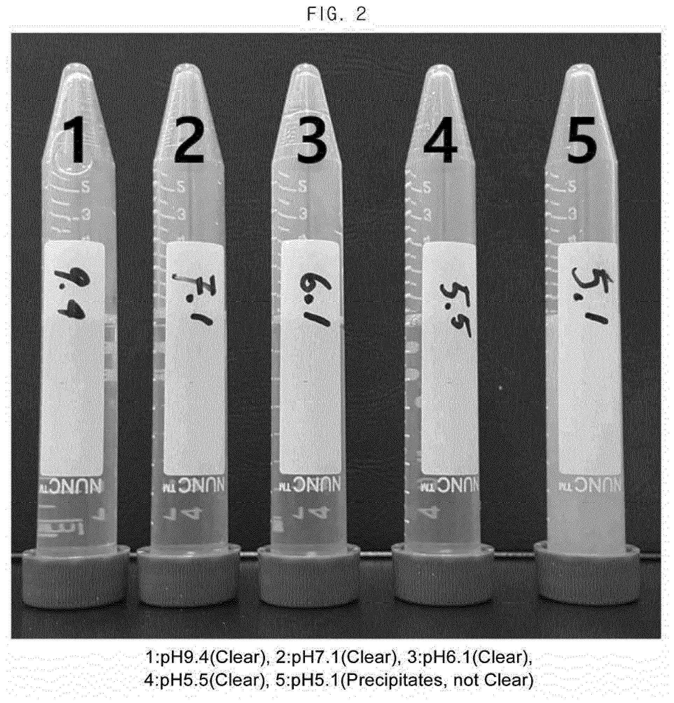

Particularly, it was prepared in accordance with the same procedure as in Example 1, except that 350 g of maltodextrin as one high molecular weight aqueous soluble starch conversion product per 17.5 g of ursodeoxycholic acid was used. 2.0 g of sodium hydroxide pellets were dissolved in 400 ml of purified water and then used. As shown in Table 3, the prepared ursodeoxycholic acid solution formed a clear aqueous solution at pH 9.4, 7.1, 6.1, and 5.5 without visual precipitation, but formed precipitates at pH 5.1. FIG. 2 is images illustrating whether a clear aqueous solution of the ursodeoxycholic acid solution is formed or not at each pH value.

**(Example 5) A clear aqueous solution with 1:25 of weight ratio of UDCA to maltodextrin**

A clear aqueous stock solution of aqueous solubilized UDCA containing natural UDCA and aqueous soluble starch having low dextrose equivalent was prepared.

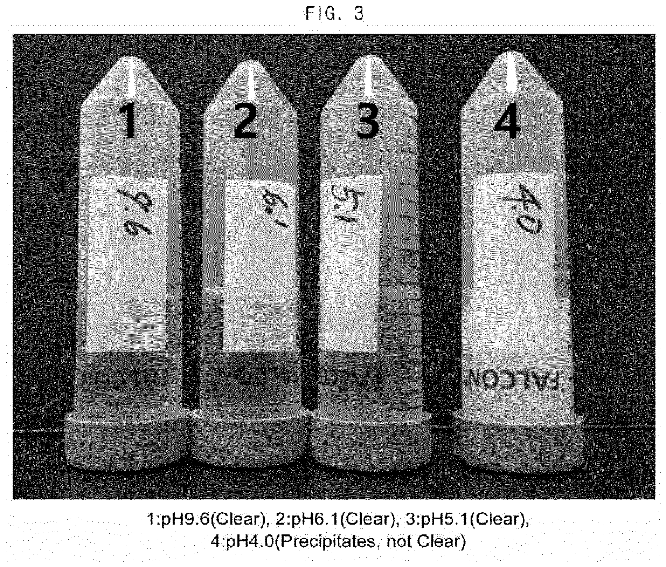

Particularly, it was prepared in accordance with the same procedure as in Example 1, except that 350 g of maltodextrin as one high molecular weight aqueous soluble starch conversion product per 14 g of ursodeoxycholic acid was used. 1.7 g of sodium hydroxide pellets were dissolved in 400 ml of purified water and then used. As shown in Table 3, the prepared ursodeoxycholic acid solution formed a clear aqueous solution at pH 9.6, 6.1, and 5.1 without visual precipitation, but formed precipitates at pH 4.0. FIG. 3 is images illustrating whether a clear aqueous solution of the ursodeoxycholic acid solution is formed or not at each pH value.

**(Example 6) A clear aqueous solution with 1:30 of weight ratio of UDCA to maltodextrin**

A clear aqueous stock solution of aqueous solubilized UDCA containing natural UDCA and aqueous soluble starch having low dextrose equivalent was prepared.

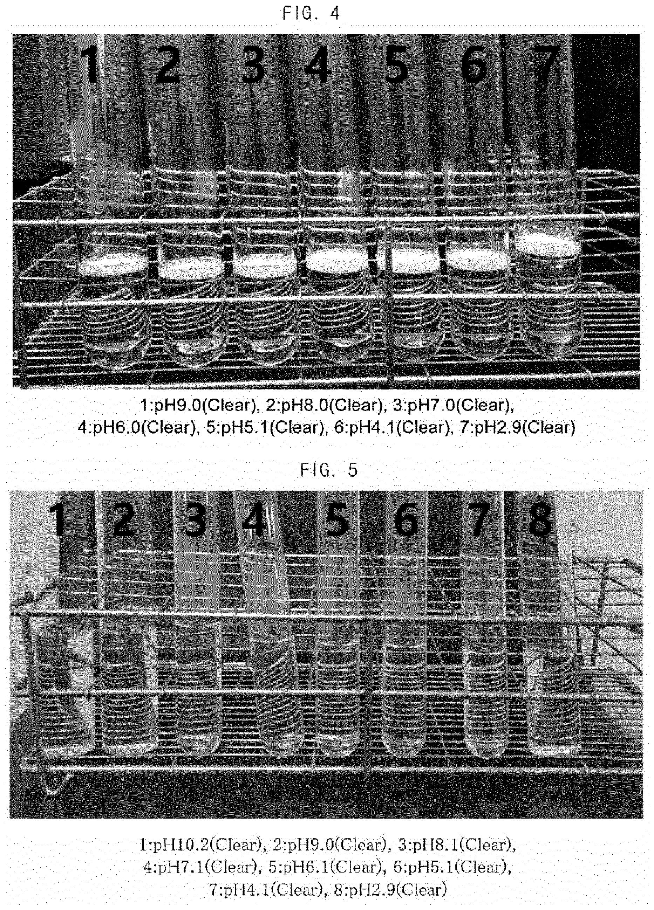

Particularly, it was prepared in accordance with the same procedure as in Example 1, except that 750 g of maltodextrin as one high molecular weight aqueous soluble starch conversion product per 25 g of ursodeoxycholic acid was used. 2.8 g of sodium hydroxide pellets were dissolved in 400 ml of purified water and then used. As shown in Table 3, the prepared ursodeoxycholic acid solution formed a clear aqueous solution at pH 9.0, 8.0, 7.0, 6.0, 5.1, 4.1, and 2.9 without visual precipitation. FIG. 4 is images illustrating whether a clear aqueous solution of the ursodeoxycholic acid solution is formed or not at each pH value.

**(Example 7) A clear aqueous solution containing UDCA/tUDCA/gUDCA and with 1:30 of weight ratio of UDCA/tUDCA/gUDCA to maltodextrin**

A clear aqueous stock solution of aqueous solubilized UDCA containing UDCA and UDCA derivatives and aqueous soluble starch having low dextrose equivalent was prepared.

Particularly, 0.3 g of sodium hydroxide pellet was dissolved in 500 ml of purified water. Then, 1.0 g of ursodeoxycholic acid, 0.5 g of tauroursodeoxycholic acid, and 0.5 g of glycoursodeoxycholic acid were dissolved in the sodium hydroxide solution while stirring at room temperature. 60 g of maltodextrin was added slowly to the clear solution while stirring. A preservative was then added in an amount appropriate for the pharmaceutical formulation to the clear solution obtained by performing ultrasonication (750W, 20kHz) at high throughput and the pH was adjusted by the dropwise addition of HCl. Purified water was added and adjusted to be a total of 1,000 ml. As shown in Table 3, the prepared ursodeoxycholic acid solution formed a clear aqueous solution at pH 10.2, 9.0, 8.1, 7.1, 6.1, 5.1, 4.1, and 2.9 without visual precipitation. FIG. 5 is images illustrating whether a clear aqueous solution of the ursodeoxycholic acid solution is formed or not at each pH value.

Table 3

| | Whether a clear aqueous solution was formed depending on the pH value of prepared aqueous solubilized UDCA according to each Example | | | | | |
|---|---|---|---|---|---|---|
| Example | Weight ratio of UDCA to maltodextrin | Amount of UDCA (g/L) | Amount of maltodextrin (g/L) | pH Value | Clarity | Remarks |
| 1 | 1:6 | 60 | 360 | 10.3 | Clear | |
| | | | | 9.2 | Clear | |
| | | | | 6.7 | Clear | |
| | | | | 5.4 | Precipitates | |
| 2 | 1:12 | 60 | 720 | 9.6 | Clear | |
| | | | | 7.3 | Clear | |
| | | | | 6.5 | Clear | |
| | | | | 6.1 | Clear | |
| | | | | 5.5 | Precipitates | |
| 3 | 1:15 | 50 | 750 | 9.5 | Clear | FIG. 1 |
| | | | | 8.9 | Clear | |
| | | | | 7.9 | Clear | |
| | | | | 7.1 | Clear | |
| | | | | 6.0 | Clear | |
| | | | | 5.5 | Precipitates | |
| 4 | 1:20 | 17.5 | 350 | 9.4 | Clear | FIG. 2 |
| | | | | 7.1 | Clear | |
| | | | | 6.1 | Clear | |
| | | | | 5.5 | Clear | |
| | | | | 5.1 | Precipitates | |
| 5 | 1:25 | 14 | 350 | 9.6 | Clear | FIG. 3 |
| | | | | 6.1 | Clear | |
| | | | | 5.1 | Clear | |
| | | | | 4.0 | Precipitates | |
| 6 | 1:30 | 25 | 750 | 9.0 | Clear | FIG. 4 |
| | | | | 8.0 | Clear | |
| | | | | 7.0 | Clear | |
| | | | | 6.0 | Clear | |
| | | | | 5.1 | Clear | |
| | | | | 4.1 | Clear | |
| | | | | 2.9 | Clear | |

(continued)

| Whether a clear aqueous solution was formed depending on the pH value of prepared aqueous solubilized UDCA according to each Example | | | | | | |
|---|---|---|---|---|---|---|
| Example | Weight ratio of UDCA to maltodextrin | Amount of UDCA (g/L) | Amount of maltodextrin (g/L) | pH Value | Clarity | Remarks |
| 7 | 1:30 | UDCA: 1.0g tUDCA: 0.5g gUDCA: 0.5g | 60 | 10.2 | Clear | FIG. 5 |
| | | | | 9.0 | Clear | |
| | | | | 8.1 | Clear | |
| | | | | 7.1 | Clear | |
| | | | | 6.1 | Clear | |
| | | | | 5.1 | Clear | |
| | | | | 4.1 | Clear | |
| | | | | 2.9 | Clear | |

**(Example 8-12) Clear aqueous solutions of aqueous solubilized UDCA, YSB201-1, YSB201-2, YSB201-3, YSB201-4, and YSB201-5**

A stock solution of YSB201 was first prepared by dissolving UDCA (25 g) in 400 ml NaOH (2.7 g) solution. 745 g of maltodextrin was added slowly to the obtained clear solution while vigorous stirring. The pH was then adjusted to 6.8 by the addition of HCl while performing ultrasonication (750W, 20kHz) at high throughput. Pharmaceutical grade water was added to the obtained clear solution to be a total of 1,000 ml. The YSB201 stock solution was diluted with pharmaceutical grade water to have a desired UDCA concentration and sterilized by a 0.2 $\mu$m sterilizing filtration apparatus to provide YSB201-1 (Example 8), YSB201-2 (Example 9), YSB201-3 (Example 10), YSB201-4 (Example 11), and YSB201-5 (Example 12) as test samples. This filtration is important for removing impurities from the raw material or sterilization, but it is not intended to remove the granular material because the solution is already clear.

Table 4

| Samples and UDCA Concentrations | | |
|---|---|---|
| Samples | UDCA Concentration (mg/ml) | Remarks |
| YSB201-1(Ex 8) | 0.39 | For intravitreal injection |
| YSB201-2(Ex 9) | 0.78 | For intravitreal injection |
| YSB201-3(Ex 10) | 1.56 | For intravitreal injection |
| YSB201-4(Ex 11) | 12.5 | For oral administration |
| YSB201-5(Ex 12) | 25.0 | For oral administration |

**Comparative Example. Preparation of a positive control group of Eylea®**

A 10 mg/ml positive control group, Eylea®, was prepared using PBS for intravitreal administration.
Hereinafter, all test samples were stored at 4 °C.

**2. Evaluation of the effectiveness in inhibiting choroidal neovascularization of test samples YSB201 (Example 8 - Example 10) bv intravitreal injection**

Object:

This study was conducted to investigate the efficacy of an anti-angiogenic activity of test samples YSB201 through the intravitreal injection in a laser-induced choroidal neovascularization (CNV) mouse model which the characteristic of age-related macular degeneration, choroidal neovascularization, is induced (see FIG. 6).

**2-1. Intravitreal injection of the test sample**

Type, intravitreal dose and concentration of test samples administered are summarized in Table 5. The mice used in this study were 36 C57BL/6 female mice weighing 16 - 18 g/mouse. The mice were acclimatized for 6 days and then used for the test. At the first administration, the mice with about ± 20% of the total average weight were used.

The mice were pupils dilated by dropping a dilating agent, Tropherine eye drop (Hanmi Pharm. Co. Ltd.), for 10 minutes and anesthetized by intraperitoneal injection with ketamine (30 mg/kg) and xylazine hydrochloride (2.5 mg/kg). The test sample was injected three times, each with 2 $\mu$l every two days, to both eyeballs of the mice, using an Ultra-Micro Pump (syringe with 35 gauge filled with the test sample in 100 $\mu$l glass syringe).

Table 5

| Type, Intravitreal dose and Concentration | | | | | No.of mice |
|---|---|---|---|---|---|
| No | Group | Treatment | Dose ($\mu$l) | Concentration (mg/ml) | Female |
| 1 | Control | - | - | - | 6 |
| 2 | Laser-induced CNV | Negative Vehicle (1X PBS) | 2 | 1X PBS | 6 |
| 3 | | YSB201-1 | 2 | 0.39 | 6 |
| 4 | | YSB201-2 | 2 | 0.78 | 6 |
| 5 | | YSB201-3 | 2 | 1.56 | 6 |
| 6 | | Positive Vehicle (Eylea®) | 2 | 10 | 6 |

2-1-1. Fundus Fluorescein Angiography (FFA)

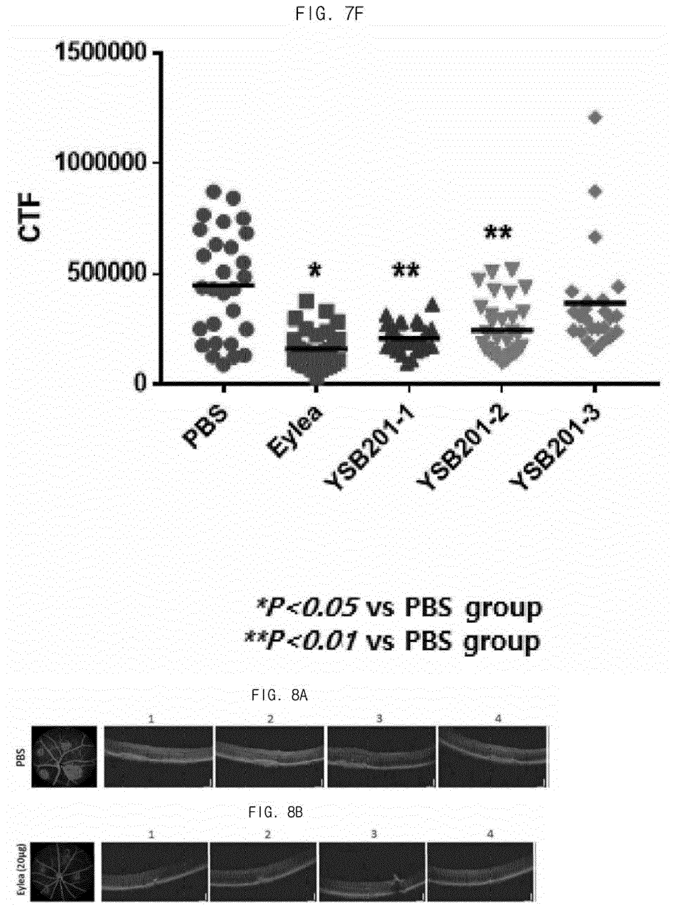

FIG. 7A - FIG. 7E are fluorescence images illustrating choroidal neovascularization generated with injection of fluorescein after 14 days of laser injury and anti-angiogenic activity with administration of test samples. FIG. 7F is a graph illustrating quantitative values thereof obtained by eliminating values exceeding $2 \times 10^6$ of the CTF calculated to correct the background. 2 $\mu$l of the positive control group, Eylea® was injected once into each eyeball. 2 $\mu$l of the test sample, YSB201 was injected into each eyeball at day 1, day 3, and day 6 after laser injury.

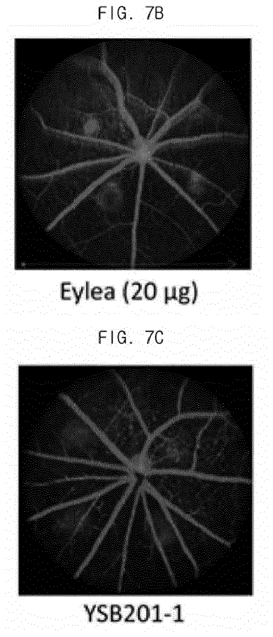

As a result of the test, the reduction effect in choroidal neovascularization was observed in the group administered with YSB201 and Eylea®. Particularly, the test samples of YSB201-1 (Example 8, FIG. 7C) and YSB201-2 (Example 9, FIG. 7D) and Eylea® (Comparative Example, FIG. 7B) statistically significantly inhibited the choroidal neovascularization (p<0.01).

2-1-2. Optical Coherence Tomography (OCT)

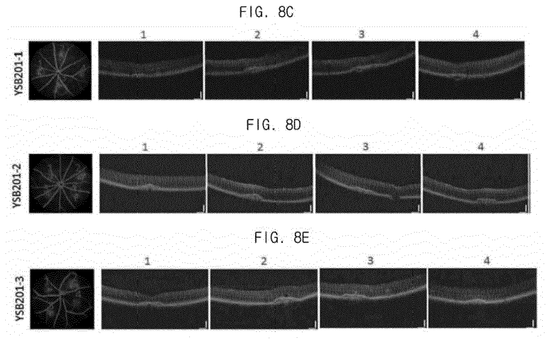

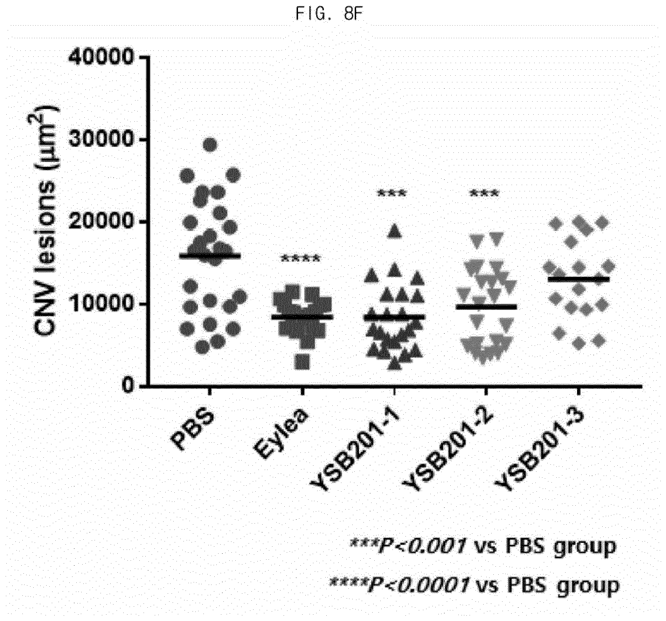

FIG. 8A - FIG. 8E are retina tomography images to observe the choroidal neovascularization and FIG. 8F is a graph that quantifies the size of CNV lesions.

As a result, choroidal neovascularization was observed in the retinas of all mice at day 14 after laser injury. CNV lesions were observed to be smaller in the group administered with YSB201 (Example 8 - Example 10) directly to the eyeballs compared with the control group administered with PBS. Particularly, the test samples YSB201-1 (Example 8, FIG. 8C) and YSB201-2 (Example 9, FIG. 8D) and Eylea® (FIG. 8B) statistically significantly inhibited choroidal neovascularization (p<0.001).

2-1-3. Electroretinography (ERG)

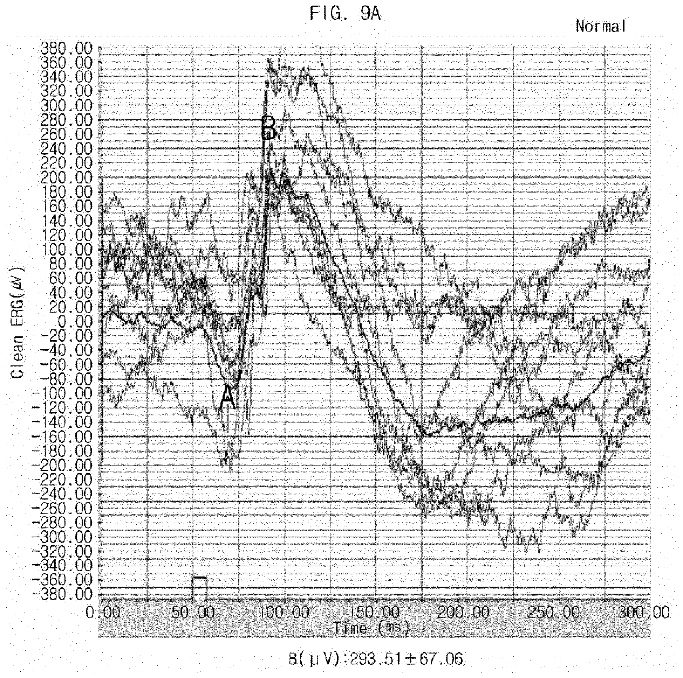

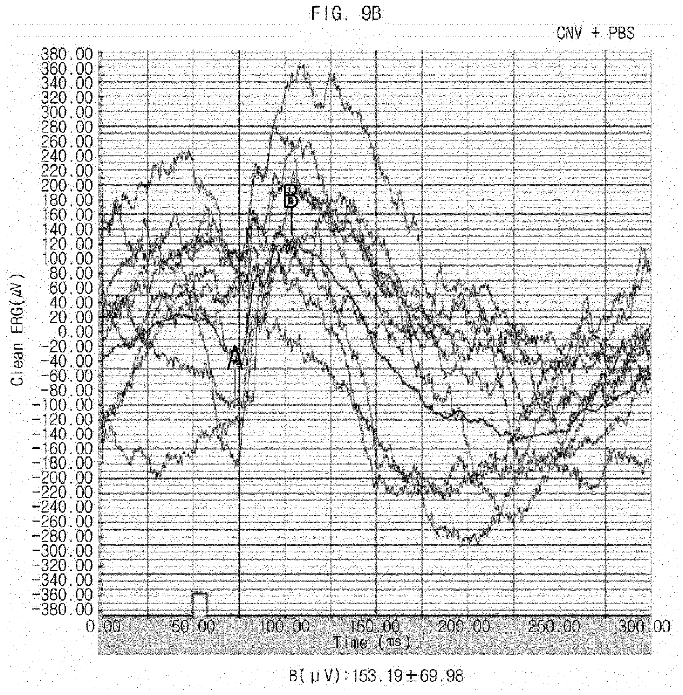

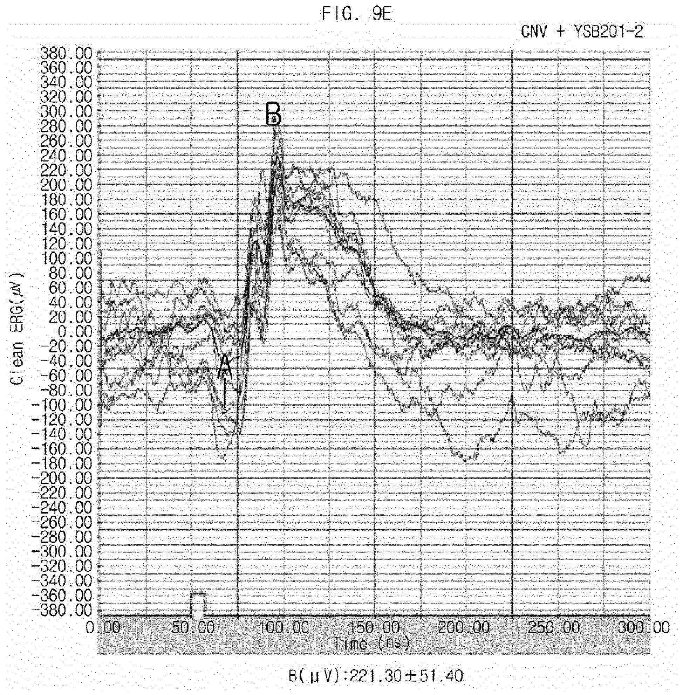

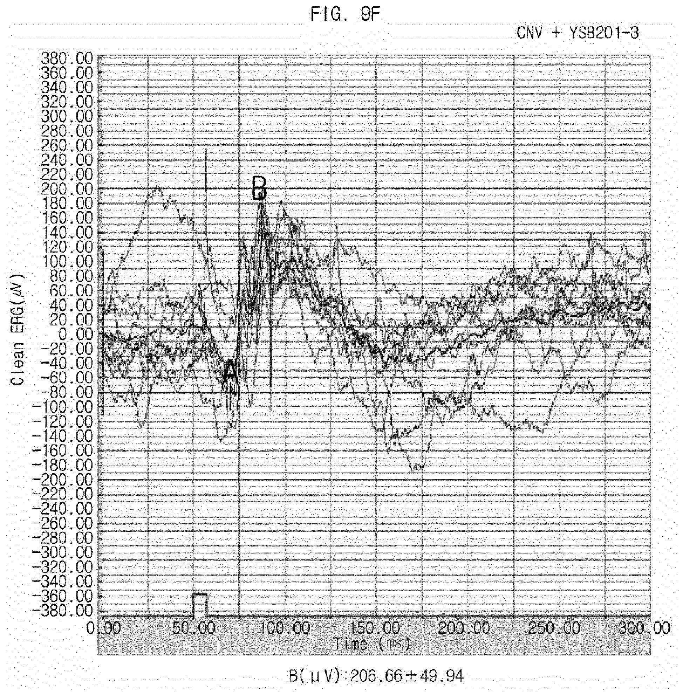

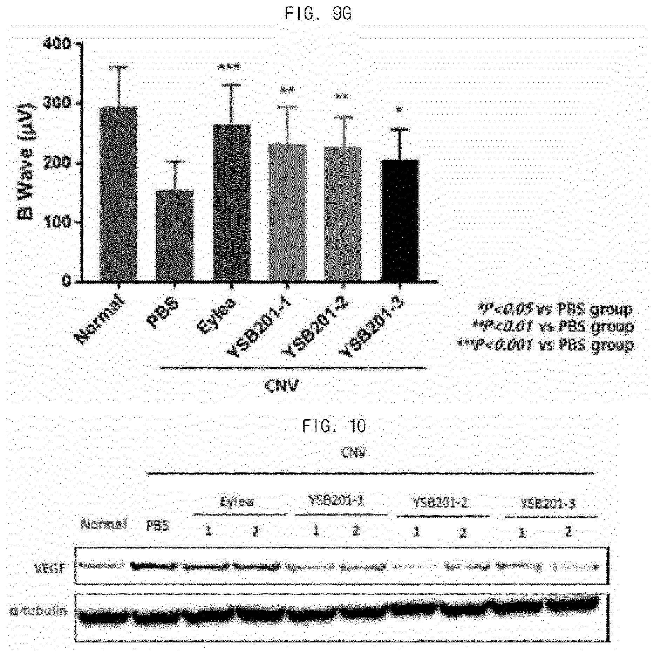

FIG. 9A - FIG. 9F are the test results that measured the response degree of retina to the white light after dark adaptation of group with intravitreal injection at day 15 after laser injury.

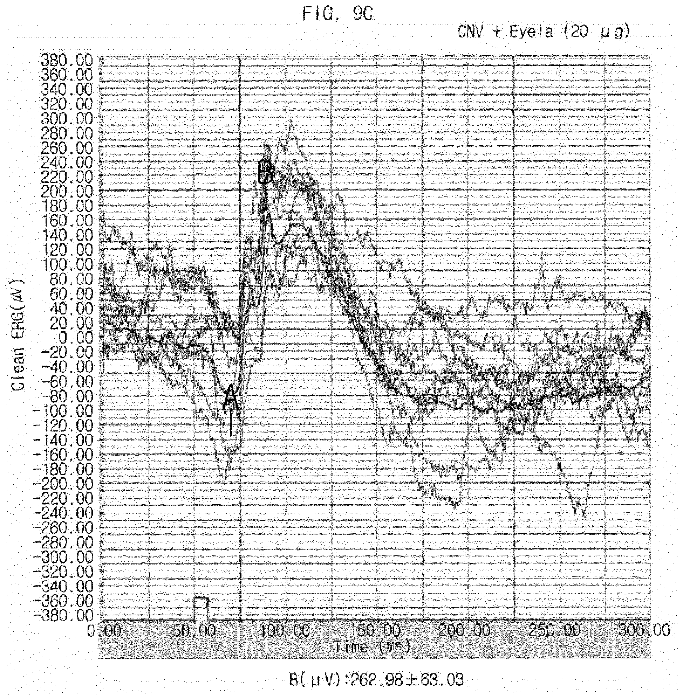

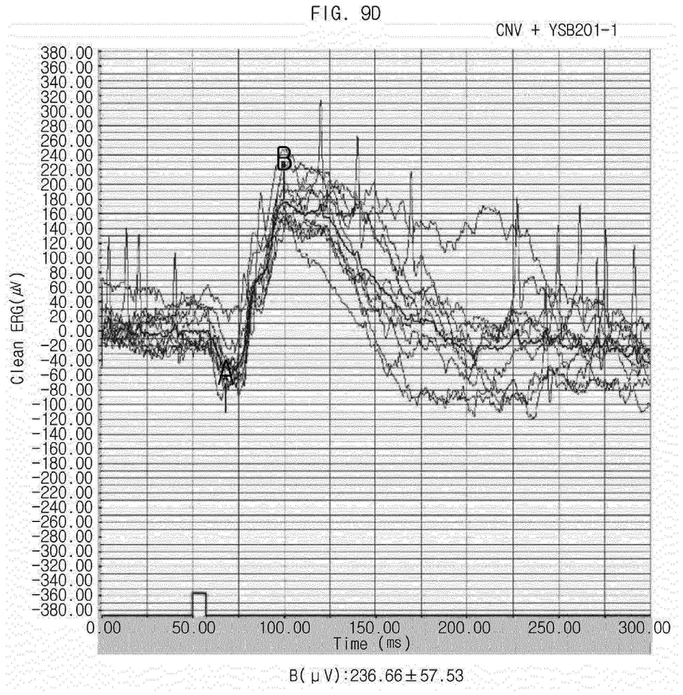

Experimental results showed that 15 days later after laser injury, the response degree to the white light was reduced because the retinal function was deteriorated due to the CNV. However, ERG response was increased with the group administered with YSB201 (Example 8 - Example 10, FIG. 9D - FIG. 9F) and Eylea® (FIG. 9C) by intravitreal injection due to recovery of the retinal function. ERG response was not statistically significant in the group administered with YSB201-3 even though it was higher compared with the group administered with PBS.

2-1-4. Western blot analysis

FIG. 10 illustrates the result of Western blot analysis that analyzes the expression of vascular endothelial growth factor (VEGF) in the choroid and the retina of the group with intravitreal administration at day 15 later after laser injury.

The expression of VEGF was significantly increased in the group administered with PBS in the eyeballs compared with the normal group, while the expression of VEGF was decreased in the group administered with YSB201 (Example 8 - Example 10) and Eylea® in the eyeballs. In detail, the expression of VEGF was decreased in the group administered with Eylea® compared with that in the group administered with PBS, but not significantly decreased compared with that in the group administered with YSB201 (Example 8 - Example 10). This suggests that Eylea® inhibits the activity of VEGF secreted from choroidal and retinal cells due to the nature of protein antibody, but it does not prevent from increasing the expression of VEGF in choroidal and retinal cells. In other words, Eylea® has a disadvantage in that it cannot securely and continuously inhibit the intracellular VEGF expression at the gene level. On the other hand, in the case of YSB201-treated group (Example 8 - Example 10), the expression level of VEGF in the choroid and retinal cells was much lower than that of Eylea. This is because YSB201 (Example 8 - Example 10) down-regulated the expression of VEGF in choroidal and retinal cells at the gene level, and securely and continuously inhibited the neovascularization unlike Eylea.

### 3. Evaluation of the effectiveness in inhibiting choroidal neovascularization of YSB201 (Example 11 and Example 12) by oral administration

Object: This study was conducted to investigate the efficacy of an anti-angiogenic activity of test samples YSB201 (Example 11 and Example 12) through oral administration which delivers UDCA to the eyeballs across the blood-retinal barrier in a laser-induced choroidal neovascularization (CNV) mouse model which the characteristic of age-related macular degeneration, choroidal neovascularization, is induced (see FIG. 11).

### 3-1. Oral administration of the test sample

Type, dose and concentration of test samples administered are summarized in Table 6. The mice used in this study were 24 C57BL/6 female mice weighing 16 - 18 g/mouse. The mice were acclimatized for 6 days and then used for the test. At the first administration, the mice with about $\pm$ 20% of the total average weight were used.

The dose of each individual was calculated based on the body weight measured immediately prior to administration and then orally administered using a disposable syringe equipped with sonde. Oral administration was provided once a day between 11:00 am and 2:00 pm from 10 days before laser injury. After laser injury, the mice were also orally administered once a day for 10 days between 11:00 am and 2:00 pm, and the mice were euthanized on day 15 to prepare measurement samples.

Table 6

| | | | Dose | | | No.of mice |
|---|---|---|---|---|---|---|
| No | Group | Treatment | mg/kg | mg/kg/day | Concentration (mg/ml) | Female |
| 1 | Control | - | - | - | - | 6 |
| 2 | Laser-induced CNV | Vehicle (oleve oil) | - | - | - | 6 |
| 3 | | YSB201-4 | 125 | 125 | 12.5 | 6 |
| 4 | | YSB201-5 | 250 | 250 | 25 | 6 |

3-1-1. Fundus Fluorescein Angiography (FFA)

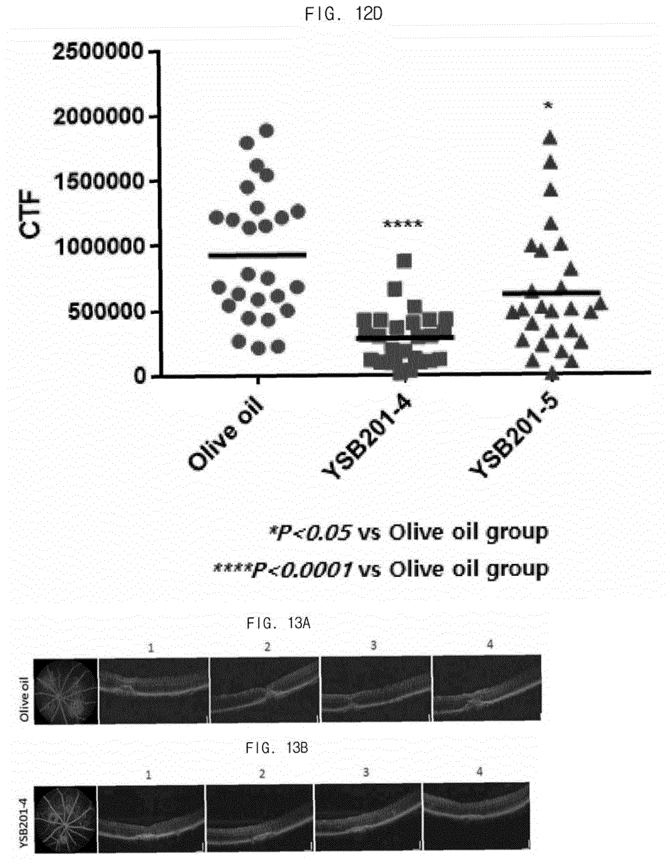

FIG. 12A - FIG. 12C are fluorescence images illustrating choroidal neovascularization generated with injection of fluorescein after 13 days of laser injury and anti-angiogenic activity with administration of test samples. FIG. 12D is a graph illustrating quantitative values thereof obtained by eliminating values exceeding $2 \times 10^6$ of the CTF calculated to correct the background. The results showed that the group orally administered with YSB201 (Example 10 and Example 11) showed a high reduction pattern in CNV lesions compared with the control group (vehicle), and YSB201-4 (125 mg/kg/day, Example 11) showed the best efficacy (p <0.001). YSB201-5 (250 mg/kg) showed a tendency to decrease CNV lesions compared with the control group, but it was not statistically significant.

3-1-2. Optical Coherence Tomography (OCT)

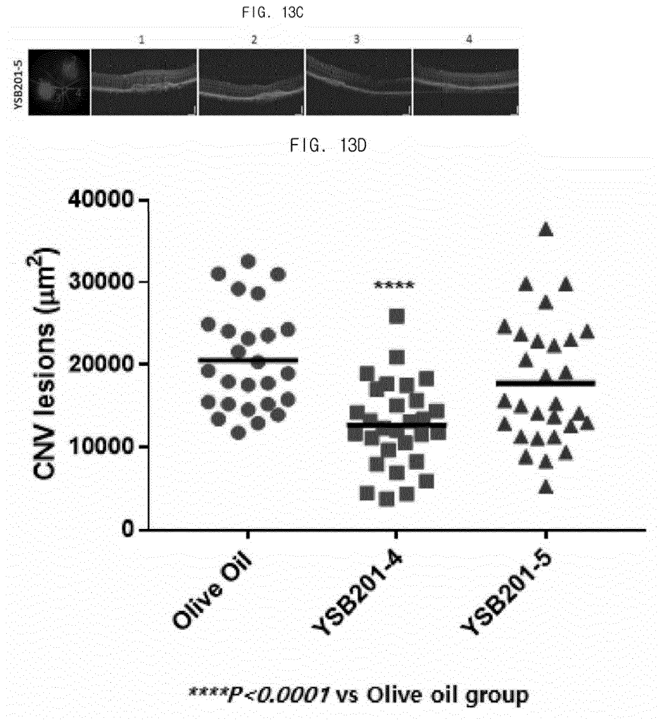

FIG. 13A - FIG. 13C are retina tomography images to observe the choroidal neovascularization and FIG. 13D is a graph that quantifies the size of the CNV lesion.
As a result, choroidal neovascularization was observed in the retinas of all mice at day 13 after laser injury. CNV lesions were observed to be much smaller in the group orally administered with YSB201-4 (Example 11) compared with the control group administered with olive oil.

3-1-3. Electroretinography (ERG)

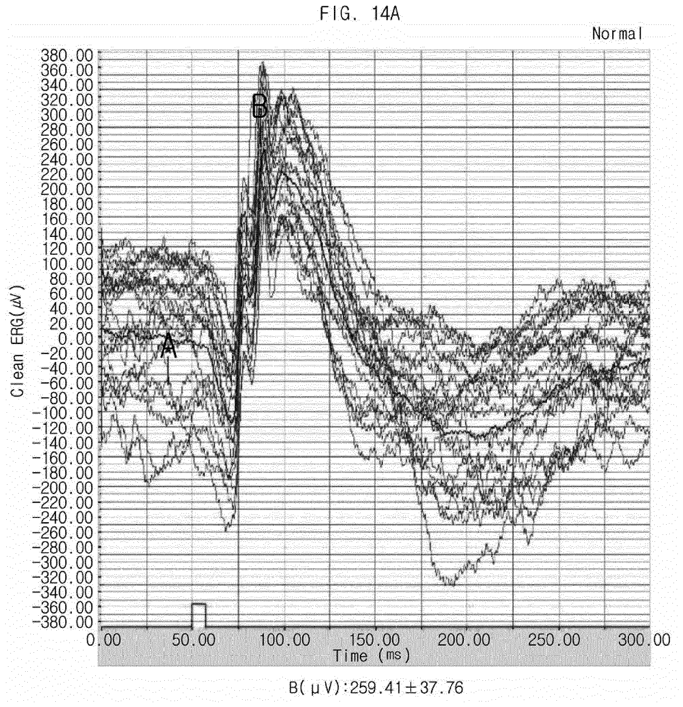

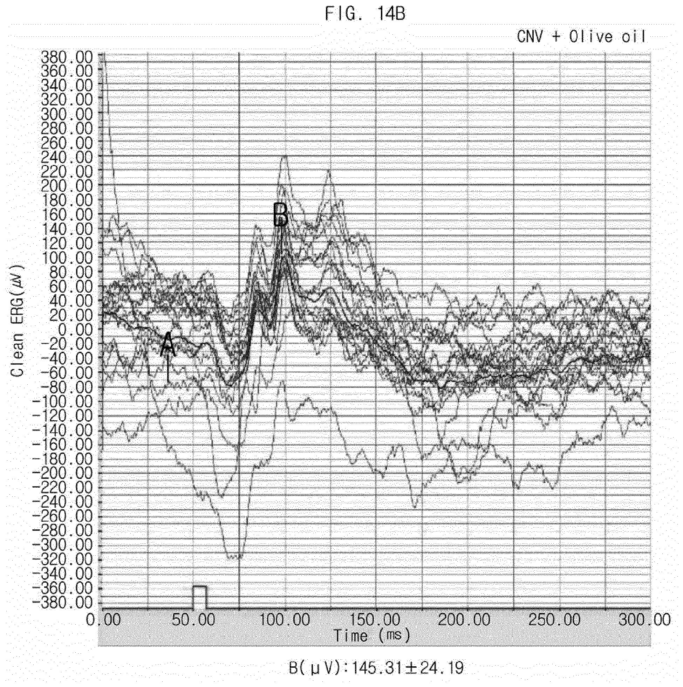

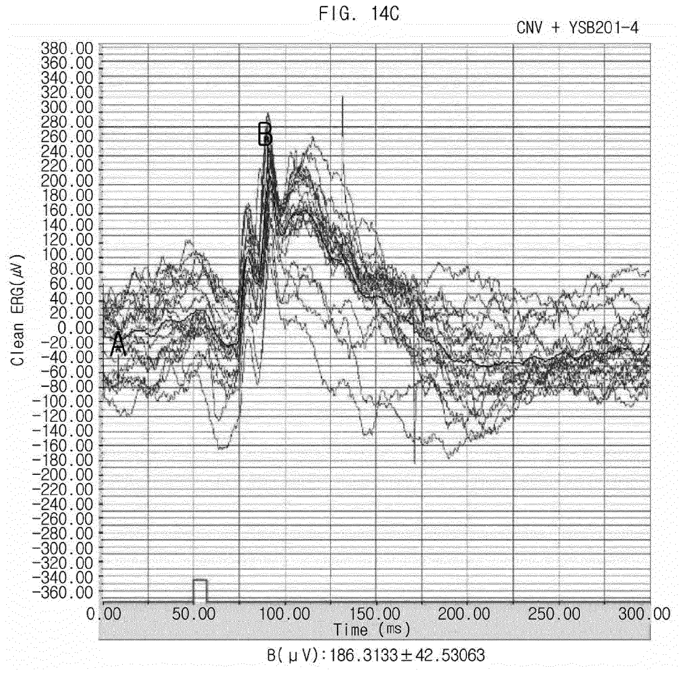

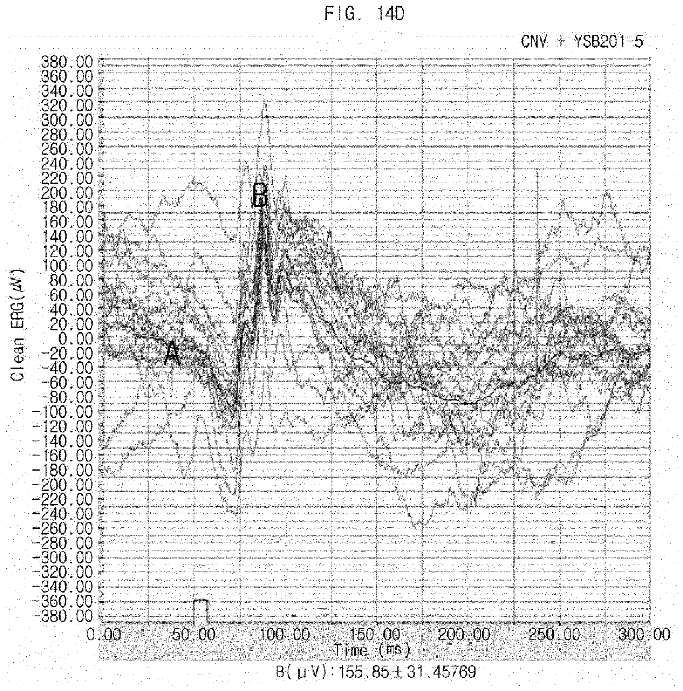

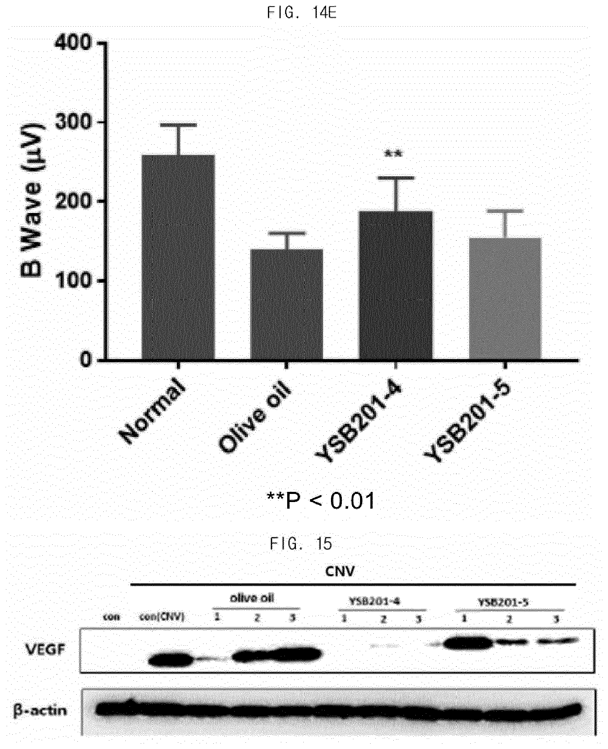

FIG. 14A - FIG. 14E are the test results that measured the response degree of retina to the white light after dark adaptation of group with oral administration at day 14 after laser injury.
Experimental results showed that at day 14 later after laser injury, the response degree to the white light was reduced because the retinal function was deteriorated due to the CNV. However, ERG response was increased in the group orally administered with YSB201-4 (Example 11, FIG. 14C) so that the retinal function was recovered up to 73% compared with the normal group, which was statistically significant (p<0.05). On the other hand, ERG response was not statistically significant in the group administered with YSB201-5 (Example 12, FIG. 14D) even though it was increased B-wave value compared with the control group.

3-1-4. Western blot analysis

FIG. 15 illustrates the result of Western blot analysis that measured the expression level of vascular endothelial growth factor (VEGF) in the choroid and the retina of the group with oral administration at day 14 after laser injury.
The expression of VEGF was increased in the group without oral administration and the group administered with olive oil, while the expression of VEGF was significantly decreased in the group orally administered with YSB201-4 (Example 11).

**Conclusions on the Inhibitory Effect of Choroidal Neovascularization**

This test was conducted to investigate the possibility of the aqueous solubilized UDCA (YSB201) in clear aqueous solution as a therapeutic agent for wet macular degeneration. It was confirmed, using the CNV mouse model that induces choroidal neovascularization associated with the wet macular degeneration, that YSB201 had the anti-angiogenic activity.
The choroidal neovascularization was induced by irradiating laser to the Bruch's membrane of a mouse to partially destroy it. Two tests were conducted to investigate preventive and therapeutic efficacies of test samples in macular degeneration, of which one test was conducted by orally administering 125 mg/Kg of YSB201-4 (Example 11) or 250 mg/Kg of YSB201-5 (Example 12) daily from 9 days before laser irradiation and the other was conducted by intravitreally injecting YSB201 directly to the eyeballs.
As a result of the intravitreal injection test, YSB201-1 (Example 8) and YSB201-2 (Example 9) were able to inhibit the choroidal neovascularization to a similar degree as the positive control group, Eylea®. No adverse reactions to the retina due to intravitreal injection were observed. When the retinal tomography was taken to determine the formation of CNV lesions, the group injected with only PBS showed the formation of CNV lesions to the extent of retinal edema and also showed partial retinal degradation. However, the CNV lesion was reduced in the group administered with YSB201 and retinal degradation was inhibited in the group administered with YSB201-1 (Example 8) or YSB201-2 (Example 9), which was confirmed in the ERG test. In the western blot study using proteins extracted from the retina, it was observed that inhibition of the expression of VEGF protein of the positive control group of Eylea® was less compared to that of YSB201.
In the oral administration test, YSB201-4 (125 mg/ml, Example 11) was found to inhibit choroidal neovascularization and further to be more potent in inhibition of the formation of CNV lesions even though the concentration was half of YSB201-5 (Example 12). In the electroretinography (ERG) to investigate the functions of the retina, a decrease in amplitude, a typical characteristic of retinal disease in CNV mice, was observed. The EGR response is separated into two components of a-wave and b-wave in which the a-wave (receptor potential) is a negative wave derived from the photoreceptor by photostimulation and reflects the function of the photoreceptors and the b-wave (Müller cell potential) is derived from Müller cells during the transmission process of the photoreceptors, resulting in a sudden positive-potential wave. In the normal retina, a-wave is negative and b-wave is positive, so the potential difference between these two waves can be used to determine the function of the retina.

It is known that a-wave and b-wave are not lost, but amplitudes thereof are decreased in the clinical macular degeneration. YSB201-4 (Example 11) shows the best efficacy and also effective reduction in the expression of VEGF. When fluorescein is intraperitoneally injected on day 13 after laser injury, it is confirmed with fundus fluorescein angiography that the CNV lesions are significantly reduced in the group administered with YSB201.

In conclusion, YSB201 is effective in reducing the CNV lesions by effectively inhibiting the expression of VEGF when administered orally or directly to the retina.

## 4. Pharmacokinetic analysis of aqueous solubilized UDCA in a clear aqueous solution in plasma and eyeballs

Object: This study was conducted to investigate whether a drug ingredient could be delivered to the plasma and sequentially into the eyeball across the blood-retinal barrier in a therapeutically active amount and to investigate pharmacokinetics in other tissues when the test sample YSB201-4 (125 mg/kg, Example 11) was orally administered to C57BL/6 mice.

4-1. Pharmacokinetic analysis

4-1-1. Plasma sample analysis

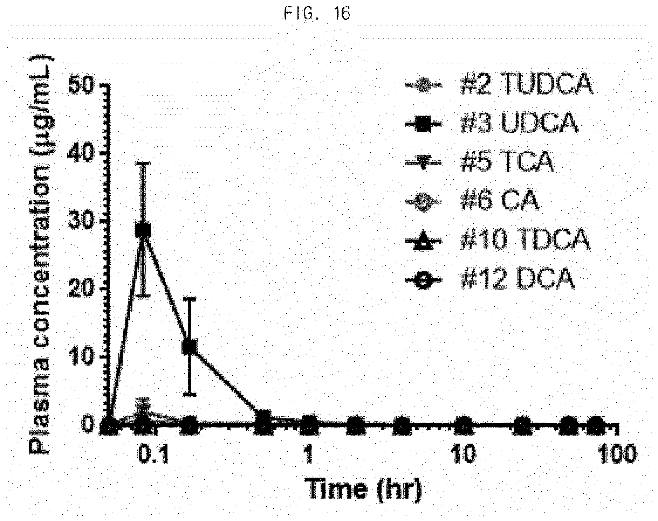

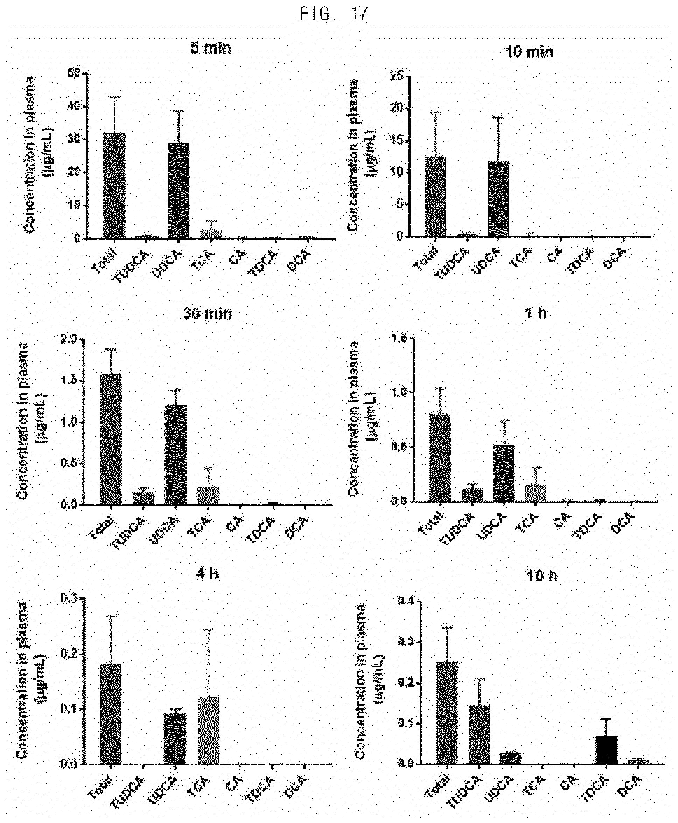

Plasma samples were analyzed after oral administration of YSB201-4 (125 mg/kg, Example 11). The concentration of UDCA in the blood of 1, 2, 3 and 4 test groups reached the maximum plasma concentration of $36.53 \pm 3.32$ (standard error value) $\mu$g/mL between 5 and 10 minutes immediately after oral administration. The concentration of UDCA in the plasma was decreased after 4 hours (FIG. 16 and FIG. 17).

4-1-2. Pharmacokinetic data analysis

Pharmacokinetics were evaluated by oral administration of YSB201-4 (125 mg/kg, Example 11) to measure pharmacokinetic parameters (Table 7). The test results showed that the time to reach the maximum blood plasma concentration was between 5 and 10 minutes and the half-life was estimated to be about 1.5 - 2 hours.

Table 7

Pharmacokinetic analysis with the plasma concentration of UDCA in the test mice

(1, 2, 4 groups) after oral administration of YSB201-4 (125 mg/kg, Example 11)

| PK parameter (Units) | Subject | | | | Arithmetic mean (SD) | Geometric mean | CV (%) | 95% CI of geometric mean | |
|---|---|---|---|---|---|---|---|---|---|
| | 1 | 2 | 3 | 4 | | | | Lower limit | Upper limit |
| Cmax (μg/mL) | 31.72 | 45.78 | 31.72 | 36.90 | 36.53 (± 3.32) | 36.11 | 18.16 | 27.39 | 47.59 |
| Tmax (hr) | 0.083 | 0.083 | - | 0.083 | 0.083 (± 0) | 0.083 | 0 | 0.08 | 0.08 |
| $AUC_{0-48}$ (hr·μg/mL) | 7.42 | 5.65 | 8.10 | 5.42 | 6.65 (± 0.657) | 6.55 | 19.76 | 4.78 | 8.98 |
| $AUC_{0-\infty}$ (hr·μg/mL) | 7.42 | 5.65 | 8.10 | 5.42 | 6.65 (± 0.657) | 6.55 | 19.76 | 4.78 | 8.98 |
| $k$ ($hr^{-1}$) | 0.58 | 0.57 | 0.56 | 0.73 | 0.384 (± 0.034) | 0.381 | 15.13 | 0.27 | 0.55 |
| $T_{1/2}$ (hr) | 1.20 | 1.21 | 1.25 | 0.96 | 1.15 (± 0.067) | 1.15 | 11.59 | 0.94 | 1.40 |
| CL/F (L/hr) | 0.36 | 0.48 | 0.33 | 0.50 | 0.418 (± 0.041) | 0.412 | 19.51 | 0.30 | 0.56 |
| V/F (L) | 0.63 | 0.83 | 0.60 | 0.69 | 0.688 (± 0.052) | 0.682 | 15.15 | 0.54 | 0.86 |
| MRT (hr) | 1.43 | 1.35 | 1.01 | 1.05 | 1.21 (± 0.107) | 1.20 | 17.63 | 0.90 | 1.59 |

\* The mean value is expressed as mean ± standard error

4-2-1. Sample analysis in the eyeball tissue

After oral administration of YSB201-4 (125 mg/kg, Example 11), the sample in the eyeball tissues of the test mice (1, 2, 3, and 4 groups) (n=4) was analyzed. The maximum concentration of UDCA in the eyeball was 8.05 ± 3.66 μg/g tissue with $T_{max}$ of 0.1 hour.

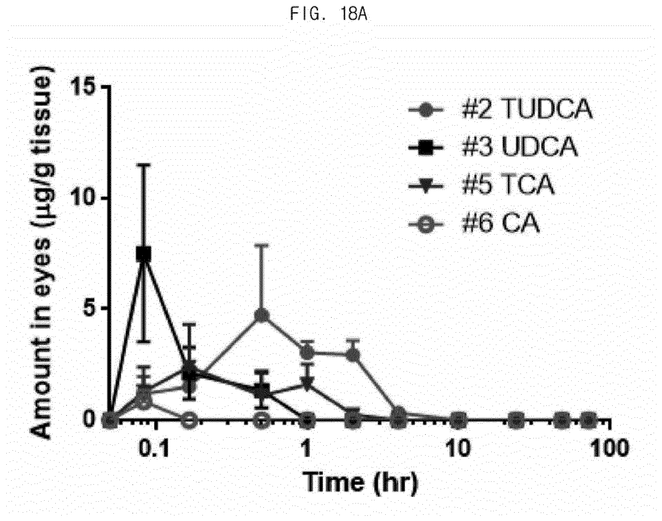

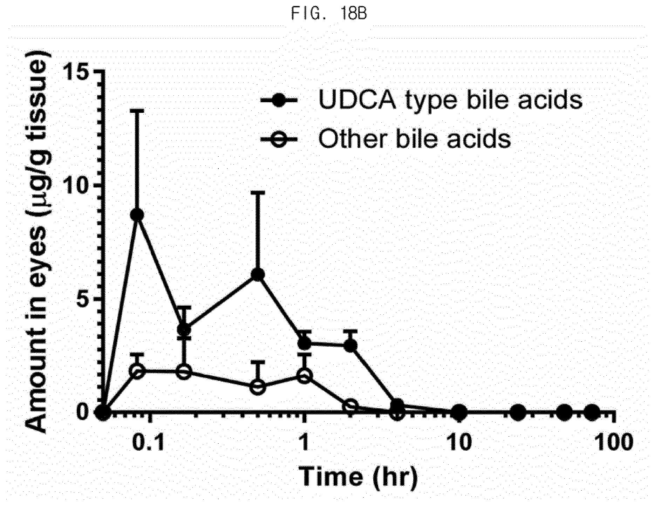

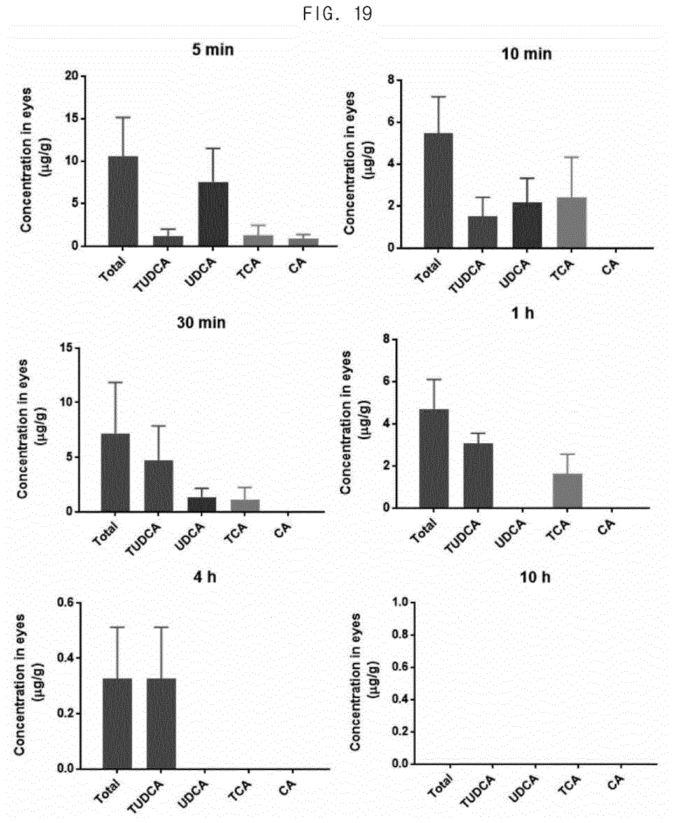

tUDCA, which is an *in vivo* metabolite of UDCA and known for impact on cell protection, was also delivered into the eyeball over time, showing the maximum concentration of 6.51 ± 2.47 μg/g tissue at $T_{max}$ of 1.38 hours (Table 8, FIG. 18A, FIG. 18B, and FIG. 19).

Table 8

Pharmacokinetic analysis with the concentration of bile acids in the eyes of the test mice (1, 2, 3, 4 groups) (n=4) after oral administration of YSB201-4 (125 mg/kg, Example 11)

<The standard error of the mean value is indicated in parentheses>

| Parameter (Units) | TUDCA | UDCA | TCA | CA |
|---|---|---|---|---|
| Cmax (μg/g tissue) | 6.51 (2.47) | 8.05 (3.66) | 4.21 (0.83) | 0.86 (0.51) |
| Tmax (hr) | 1.38 (0.38) | 0.10 (0.02) | 0.67 (0.20) | 0.04 (0.02) |

(continued)

Pharmacokinetic analysis with the concentration of bile acids in the eyes of the test mice (1, 2, 3, 4 groups) (n=4) after oral administration of YSB201-4 (125 mg/kg, Example 11)
<The standard error of the mean value is indicated in parentheses>

| Parameter (Units) | TUDCA | UDCA | TCA | CA |
|---|---|---|---|---|
| AUC (hr·$\mu$g/g tissue) | 10.41 (0.47) | 1.64 (0.60) | 2.53 (0.75) | 0.14 (0.02) |

According to FIG. 18A, FIG. 18B, and FIG. 19, if YSB201-4 (Example 11) is orally administrated, the aqueous solubilized UDCA was quickly absorbed into the blood and further delivered to the eyeballs across the blood-retinal barrier. This means that it can function effectively while staying in the eyeballs for over 2 hours. The concentration of UDCA gradually decreased after $T_{max}$, but its *in vivo* metabolite, tUDCA, was delivered and stayed for 4 hours to provide cytoprotection. And after that, the concentration of tUDCA also gradually decreased.

Thus, while the total bile acids stayed in the eyeballs for 4 hours, the sum of the concentrations of UDCA-based bile acids (UDCA, TUDCA, and GUDCA) that provide cytoprotection were always higher than the sum of other bile acids(e.g., TCA, CA) that have surfactant function. This means that the cytoprotective function can inhibit the surfactant function to protect retinal cells.

Therefore, the oral administration of YSB201-4 (Example 11) allows delivering UDCA and UDCA-based bile acids to the eyeballs at high concentrations to inhibit choroidal neovascularization and simultaneously recover the damaged retinal cells effectively.

4-3-1. Analysis of samples in stomach tissues

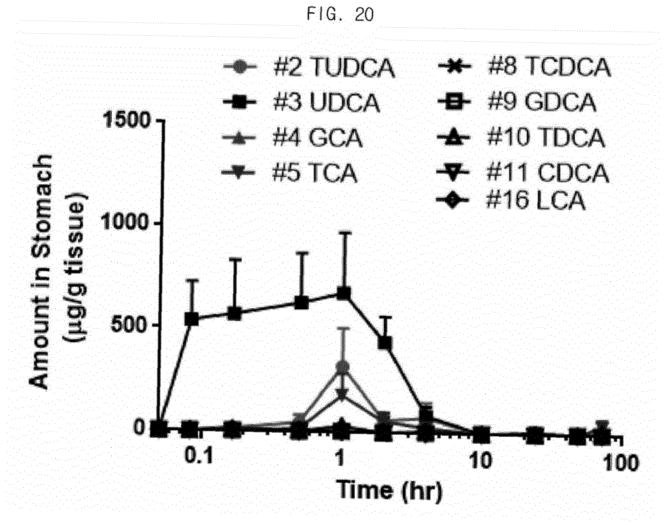

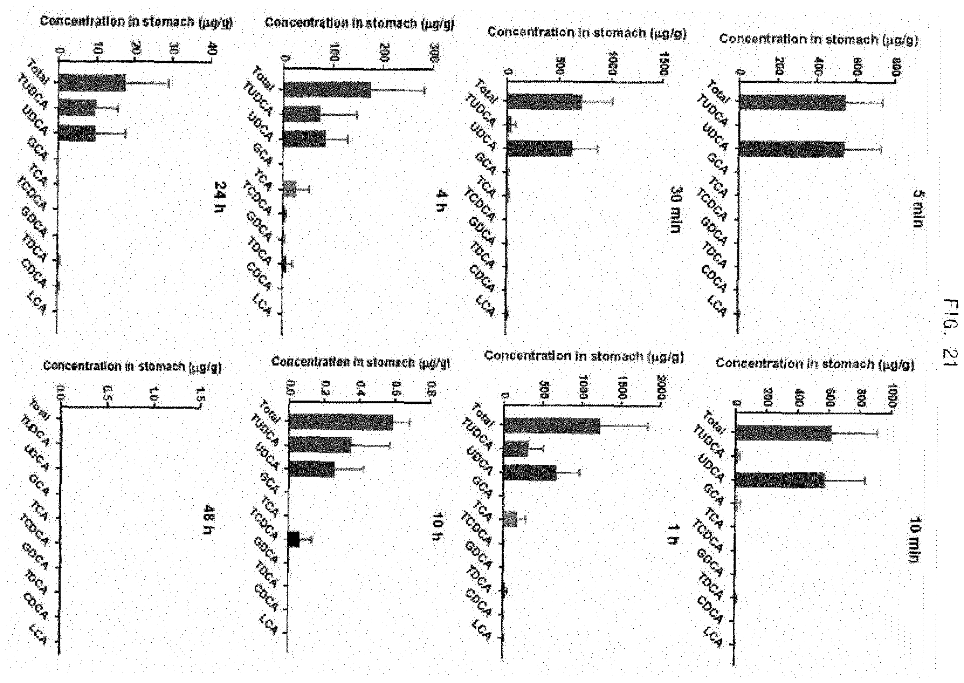

The stomachs of the test mice were analyzed after homogenously fragmented and extracted under the same analytical conditions as in 4-1-1. The concentration of UDCA in the stomach of the test mice of 1, 2, 3, and 4 groups was increased rapidly after 5 minutes and disappeared after 4 hours after oral administration of the test sample (FIG. 20 and FIG. 21).

4-4-1. Pharmacokinetic data analysis of each tissue sample

Pharmacokinetic analysis of the concentration of UDCA in plasma, eyeballs and gastrointestinal tissues (liver, stomach, small intestine, large intestine) of the test mice of 1, 2, 3, and 4 groups was performed after oral administration of YSB201-4 (125 mg/kg, Example 11) (Table 9). The maximum concentration ($C_{max}$) of UDCA in eyeballs was about 0.2 times of UDCA $C_{max}$ in the plasma, and the $C_{max}$ in liver and stomach was about 1.6 - 28 times of UDCA $C_{max}$ in the plasma. The reason why $T_{max}$ in plasma is as fast as 0.083 hour is because $T_{max}$ in liver and stomach is fast (0.1 h, 0.71 h) (Table 9)

Table 9

Pharmacokinetic analysis with the concentration of UDCA in the plasma and the tissues of the test mice (1, 2, 3, 4 groups) (n=4) after oral administration of YSB201-4 (125 mg/kg, Example 11)

| Parameter (Units) | plasma | eyes | liver | stomach | small intestine | large intestine |
|---|---|---|---|---|---|---|
| Cmax ($\mu$g/g tissue) | 36.56 (3.30) | 8.05 (3.66) | 56.00 (28.0) | 896.5 (222.6) | 857.3 (111.4) | 32.16 (10.65) |
| Tmax (hr) | 0.083 (0.0) | 0.10 (0.02) | 0.10 (0.02) | 0.71 (0.44) | 1.88 (0.77) | 1.63 (0.38) |
| AUC (hr-$\mu$g/g tissue) | 6.67 (0.65) | 1.64 (0.60) | 141.1 (33.0) | 2113 (584.1 ) | 6165 (2007) | 218.2 (73.2) |
| AUC ratio | 2.30 | 4.51 | 14.49 | 2.44 | 15.13 | 1.26 |

The standard error of the mean value is indicated in parentheses.

Units of AUC and the concentration of UDCA in plasma are hr·$\mu$g/mL and $\mu$g/mL, respectively.

4-5-1. Changes in UDCA-based bile acids and other bile acids

The total concentration of UDCA and UDCA-based bile acids, which are tUDCA and gUDCA produced by *in*

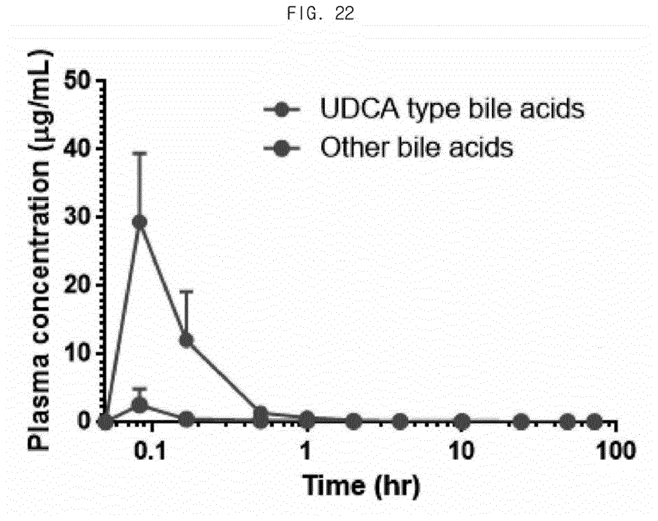

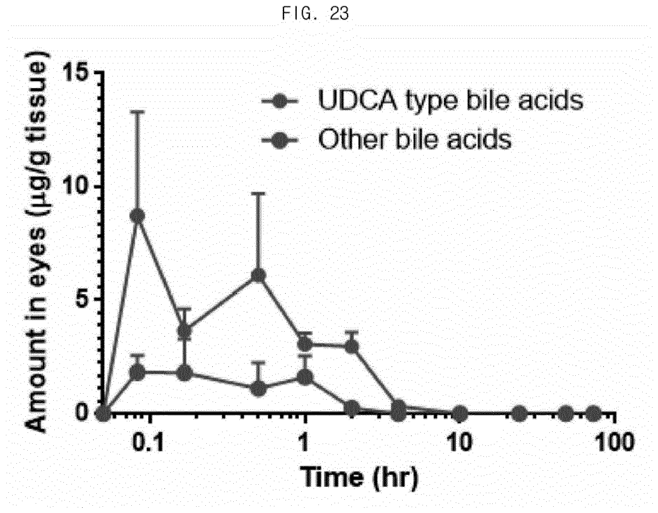

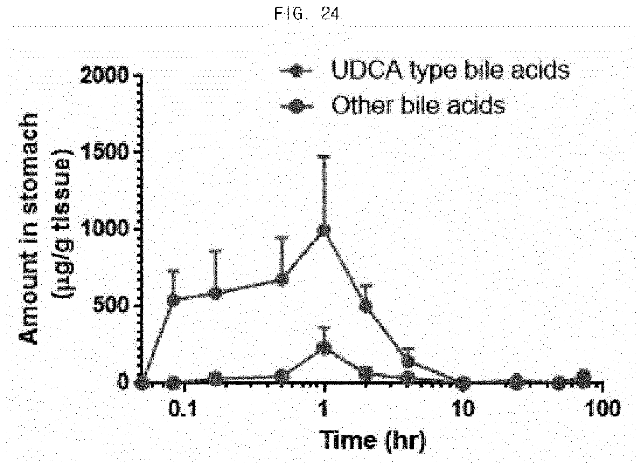

*vivo* metabolism after oral administration of YSB201, was sharply increased in plasma, eyeballs and stomach tissues. The concentration of these UDCA and UDCA-based bile acids was significantly higher than that of other bile acids (FIG. 22 - FIG. 24).

**Conclusion of pharmacokinetic analysis**

Oral administration of the aqueous solubilized UDCA in a clear solution (YSB201) allows delivering UDCA to plasma at high concentration and sequentially delivering therapeutic active amount of UDCA to the eyeballs across the blood-retinal barrier. Since UDCA in the eyeballs does not disappear immediately but stays for about 2 hours to provide its effects and sequentially tUDCA, which is a metabolite of UDCA, is delivered in the eyeballs and stays for about 4 hours to continuously provide its effects, oral administration of YSB201 can effectively be used for the prevention and treatment of macular degeneration.

The spirit of the present disclosure has been described by way of example hereinabove, and the present disclosure may be variously modified, altered, and substituted by those skilled in the art to which the present disclosure pertains without departing from essential features of the present disclosure. Accordingly, the exemplary embodiments disclosed in the present disclosure and the accompanying drawings do not limit but describe the spirit of the present disclosure, and the scope of the present disclosure is not limited by the exemplary embodiments and accompanying drawings. The scope of the present disclosure should be interpreted by the following claims and it should be interpreted that all spirits equivalent to the following claims fall within the scope of the present disclosure.

**Claims**

1.  A composition for the prevention or the treatment of visual impairments comprising active ingredients of :

    (a) ursodeoxycholic acid (UDCA);
    (b) an aqueous soluble starch conversion product; and
    (c) water,
    wherein the composition comprises a clear aqueous solution of an aqueous solubilized ursodeoxycholic acid for all pH values.

2.  The composition of claim 1, wherein the composition is prepared for an intravitreal injection, delivering the UDCA to the retina without causing skin irritation or inflammation in the eye after intravitreal injection.

3.  The composition of claim 2, wherein a single dose of the UDCA for the intravitreal injection is 50 - 100 μl at a concentration of 0.1 - 1.5 mg/ml.

4.  The composition of claim 1, wherein the composition is prepared for an oral administration, delivering the UDCA to the blood and then further delivering the UDCA in a therapeutically active amount thereof to the eyeball across the blood-retinal barrier.

5.  The composition of claim 4, wherein a daily dose of the UDCA of the composition for the oral administration is 5 - 30 mg/kg.

6.  The composition of claim 4, wherein the composition is administered at least once a day for 20 days or more.

7.  The composition of claim 1, wherein the composition is prepared for an intravenous injection and is administered directly to the blood without blocking blood vessels and causing skin irritation.

8.  The composition of claim 1, wherein the composition is administered as an eye drop.

9.  The composition of any one of claim 1 to claim 8, wherein the visual impairment is one selected from macular degeneration, glaucoma, and diabetic retinopathy.

10. The composition of any one of claim 1 to claim 8, wherein the visual impairment is macular degeneration.

11. The composition of any one of claim 1 to claim 8, wherein the visual impairment is wet age-related macular degeneration.

12. The composition of any one of claim 1 to claim 8, wherein the composition has at least one of functions of inhibiting the development of choroidal neovascularization, promoting the recovery of retinal function, and regulating the expression level of vascular endothelial growth factor (VEGF).

13. The composition of any one of claim 1 to claim 8, wherein the ursodeoxycholic acid is an aqueous solubilized ursodeoxycholic acid selected from an aqueous soluble ursodeoxycholic acid, an aqueous soluble ursodeoxycholic acid derivative, an ursodeoxycholic acid salt, and a ursodeoxycholic acid conjugated with an amine.

14. The composition of any one of claim 1 to claim 8, wherein the ursodeoxycholic acid is at least one aqueous solubilized ursodeoxycholic acid selected from an ursodeoxycholic acid, a tauroursodeoxycholic acid and a glycoursodeoxycholic acid.

15. The composition of any one of claim 1 to claim 8, wherein the ursodeoxycholic acid is present in a therapeutically active amount.

16. The composition of any one of claim 1 to claim 8, wherein the ursodeoxycholic acid is included in an amount of 0.01 - 5 parts by weight based on the total weight of the composition.

17. The composition of claim 11, wherein the ursodeoxycholic acid is included in an amount of 0.04 - 0.16 parts by weight based on the total weight of the composition.

18. The composition of any one of claim 1 to claim 8, wherein the aqueous soluble starch conversion product is maltodextrin and the maltodextrin is contained in an amount of 1 - 70 parts by weight based on the total weight of the composition.

19. The composition of any one of claim 1 to claim 8, wherein the pH value of the composition is 3 - 9, and the aqueous soluble starch conversion product is maltodextrin, and the minimum weight ratio of the ursodeoxycholic acid to the maltodextrin is 1:16 - 1:30.

20. The composition of any one of claim 1 to claim 8, wherein the pH value of the composition is 6.5 - 8, and the aqueous soluble starch conversion product is maltodextrin, and the minimum weight ratio of the ursodeoxycholic acid to the maltodextrin is 1:13 - 1:30.

21. The composition of any one of claim 1 to claim 8, wherein the aqueous soluble starch conversion product is at least one selected from maltodextrin, dextrin, liquid glucose, corn syrup solid, soluble starch, dextran, guar gum, pectin and soluble non-starch polysaccharide.

22. The composition of any one of claim 1 to claim 8, wherein the composition is formulated into a syrup form, a cream form, a paste form, or a dried form.

23. The composition of any one of claim 1 to claim 8, wherein the composition is co-administered with a therapeutic agent for macular degeneration.

24. The composition of claim 23, wherein the therapeutic agent for macular degeneration is an anti-vascular endothelial growth factor antibody.

25. The composition of claim 24, wherein the therapeutic agent for macular degeneration is prepared for an intravitreal injection.

FIG. 1

1:pH9.5(Clear), 2:pH8.9(Clear), 3:pH7.9(Clear),
4:pH7.1(Clear), 5:pH6.0(Clear), 6:pH5.5(Precipitates, not Clear)

FIG. 2

1:pH9.4(Clear), 2:pH7.1(Clear), 3:pH6.1(Clear),
4:pH5.5(Clear), 5:pH5.1(Precipitates, not Clear)

FIG. 3

1:pH9.6(Clear), 2:pH6.1(Clear), 3:pH5.1(Clear),
4:pH4.0(Precipitates, not Clear)

FIG. 4

1:pH9.0(Clear), 2:pH8.0(Clear), 3:pH7.0(Clear),
4:pH6.0(Clear), 5:pH5.1(Clear), 6:pH4.1(Clear), 7:pH2.9(Clear)

FIG. 5

1:pH10.2(Clear), 2:pH9.0(Clear), 3:pH8.1(Clear),
4:pH7.1(Clear), 5:pH6.1(Clear), 6:pH5.1(Clear),
7:pH4.1(Clear), 8:pH2.9(Clear)

FIG. 6

C57BL/6
(Female, 7 W, 16-18 g)

Laser injury   (200 mW, 70 ms duration)

-7          0          3          5                                    14      15      (Days)

IVT

PBS (vehicle)
Eylea   (20 mg/mL, Day 0)
YSB201-1 (0.39 mg/mL/day)
YSB201-2 (0.78 mg/mL/day)
YSB201-3  (1.56 mg/mL/day)

Angiography

ERG
sampling

FIG. 7A

PBS

FIG. 7B

**Eylea (20 μg)**

FIG. 7C

**YSB201-1**

FIG. 7D

YSB201-2

FIG. 7E

YSB201-3

FIG. 7F

*P<0.05 vs PBS group
**P<0.01 vs PBS group

FIG. 8A

FIG. 8B

FIG. 8C

FIG. 8D

FIG. 8E

FIG. 8F

***P<0.001 vs PBS group

****P<0.0001 vs PBS group

FIG. 9A

B(μV):293.51±67.06

FIG. 9B

CNV + PBS

B(μV):153.19±69.98

## FIG. 9C

CNV + Eyela (20 μg)

B(μV):262.98±63.03

EP 3 581 185 A1

## FIG. 9D

CNV + YSB201-1

B(μV):236.66±57.53

43

FIG. 9E

CNV + YSB201-2

B(μV):221.30±51.40

FIG. 9F

CNV + YSB201-3

B(μV):206.66±49.94

FIG. 9G

*P<0.05 vs PBS group
**P<0.01 vs PBS group
***P<0.001 vs PBS group

FIG. 10

## FIG. 11

C57BL/6
(Female, 7 W, 16-18 g)

Laser injury (200 mW, 100 ms duration)

-10    0                                    10        13    14    (Days)

P.O

ERG
sampling

Angiography

Vehicle (Olive oil)

YSB201-4 (125 mg/Kg/day)

YSB201-5 (250 mg/Kg/day)

## FIG. 12A

olive oil

FIG. 12B

YSB201-4

FIG. 12C

YSB201-5

FIG. 12D

*P<0.05 vs Olive oil group

****P<0.0001 vs Olive oil group

FIG. 13A

FIG. 13B

FIG. 13C

FIG. 13D

****P<0.0001 vs Olive oil group

## FIG. 14A

B(μV):259.41±37.76

FIG. 14B

CNV + Olive oil

B(μV):145.31±24.19

FIG. 14C

FIG. 14D

CNV + YSB201-5

B(μV):155.85±31.45769

FIG. 14E

**P < 0.01

FIG. 15

FIG. 16

FIG. 17

FIG. 18A

FIG. 18B

FIG. 19

FIG. 20

FIG. 21

FIG. 22

FIG. 23

FIG. 24

## INTERNATIONAL SEARCH REPORT

| International application No. |
|---|
| **PCT/KR2018/001770** |

**A. CLASSIFICATION OF SUBJECT MATTER**

*A61K 31/575(2006.01)i, A61K 31/718(2006.01)i, A61K 45/06(2006.01)i, A61K 9/00(2006.01)i, A61K 9/08(2006.01)i*

According to International Patent Classification (IPC) or to both national classification and IPC

**B. FIELDS SEARCHED**

Minimum documentation searched (classification system followed by classification symbols)
A61K 31/575; A61K 31/4743; A61K 31/56; A61P 25/28; A61K 9/08; A61K 31/718; A61K 45/06; A61K 9/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: ursodeoxycholic acid(UDCA), aqueous solubilized starch, water, aqueous solution, aqueous solubilized, composition for visual disturbance illness prevention/treatment

**C. DOCUMENTS CONSIDERED TO BE RELEVANT**

| Category* | Citation of document, with indication, where appropriate, of the relevant passages | Relevant to claim No. |
|---|---|---|
| Y | KR 10-2007-0053238 A (YOO, Seo Hong) 23 May 2007<br>See paragraphs [0008], [0025], [0034]-[0036], [0110], [0113]; example 1; and claims 1, 11, 14, 19. | 1-25 |
| Y | KR 10-2008-0012258 A (REGENTS OF THE UNIVERSITY OF MINNESOTA et al.) 11 February 2008<br>See paragraphs [0017], [0019], [0022], [0027], [0030], [0032]; and claims 1, 2, 4. | 1-25 |
| Y | KR 10-2001-0074748 A (YOO, Seo Hong) 09 August 2001<br>See example III; and claims 1, 2, 11, 17, 21, 27. | 1-25 |
| Y | US 2014-0323455 A1 (VAVVAS, Demetrios G.) 30 October 2014<br>See paragraphs [0015], [0042], [0045]; and claims 1, 2. | 1-25 |
| Y | LEE, Seung Yong et al., "Comparison of Physicochemical Properties between Ursodeoxycholic Acid and Chenodeoxycholic Acid Inclusion Complexes with P-Cydodextrin", Yakhak Hoeji, 1994, vol. 38, no. 3, pages 300-310<br>See the entire document. | 1-25 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 JUNE 2018 (07.06.2018) | **07 JUNE 2018 (07.06.2018)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

| | International application No. |
|---|---|
| | **PCT/KR2018/001770** |

| Patent document cited in search report | Publication date | Patent family member | Publication date |
|---|---|---|---|
| KR 10-2007-0053238 A | 23/05/2007 | AT 458489 T | 15/03/2010 |
| | | AU 2005-279961 A1 | 09/03/2006 |
| | | AU 2005-279961 B2 | 13/05/2010 |
| | | BR PI0514725 A | 24/06/2008 |
| | | CA 2577268 A1 | 09/03/2006 |
| | | CN 101035541 A | 12/09/2007 |
| | | CN 101035541 B | 30/05/2012 |
| | | EP 1789057 A2 | 30/05/2007 |
| | | EP 1789057 B1 | 24/02/2010 |
| | | ES 2339790 T3 | 25/05/2010 |
| | | IL 181434 A | 31/05/2012 |
| | | JP 2008-511651 A | 17/04/2008 |
| | | KR 10-1414810 B1 | 18/07/2014 |
| | | RU 2007-106563 A | 10/10/2008 |
| | | RU 2428987 C2 | 20/09/2011 |
| | | US 2006-0051319 A1 | 09/03/2006 |
| | | US 8173627 B2 | 08/05/2012 |
| | | WO 2006-026555 A2 | 09/03/2006 |
| | | WO 2006-026555 A3 | 13/07/2006 |
| KR 10-2008-0012258 A | 11/02/2008 | EP 1871385 A1 | 02/01/2008 |
| | | JP 2008-530100 A | 07/08/2008 |
| | | US 2008-0194531 A1 | 14/08/2008 |
| | | WO 2006-086452 A1 | 17/08/2006 |
| KR 10-2001-0074748 A | 09/08/2001 | AU 2005-295541 A1 | 27/04/2006 |
| | | AU 2005-295541 B2 | 17/02/2011 |
| | | CA 2338457 A1 | 03/02/2000 |
| | | CA 2338457 C | 17/11/2009 |
| | | CA 2406930 A1 | 09/08/2001 |
| | | CA 2406930 C | 15/02/2011 |
| | | CA 2584184 A1 | 27/04/2006 |
| | | CN 100558407 C | 11/11/2009 |
| | | CN 101039699 A | 19/09/2007 |
| | | CN 101039699 B | 27/07/2011 |
| | | CN 101378769 A | 04/03/2009 |
| | | CN 1205922 C | 15/06/2005 |
| | | CN 1348360 A | 08/05/2002 |
| | | CN 1450914 A | 22/10/2003 |
| | | EP 1113785 A2 | 11/07/2001 |
| | | EP 1113785 B1 | 13/04/2005 |
| | | EP 1255566 A2 | 13/11/2002 |
| | | EP 1809330 A2 | 25/07/2007 |
| | | EP 1809330 B1 | 27/04/2011 |
| | | JP 2002-522357 A | 23/07/2002 |
| | | JP 2004-500378 A | 08/01/2004 |
| | | JP 2008-516977 A | 22/05/2008 |
| | | JP 2009-511577 A | 19/03/2009 |
| | | JP 2010-503667 A | 04/02/2010 |

Form PCT/ISA/210 (patent family annex) (January 2015)

INTERNATIONAL SEARCH REPORT
Information on patent family members

International application No.

**PCT/KR2018/001770**

| Patent document cited in search report | Publication date | Patent family member | Publication date |
|---|---|---|---|
| | | JP 2011-021031 A | 03/02/2011 |
| | | JP 4790123 B2 | 12/10/2011 |
| | | KR 10-0524358 B1 | 26/10/2005 |
| | | KR 10-2007-0084211 A | 24/08/2007 |
| | | KR 10-2008-0063401 A | 03/07/2008 |
| | | US 2002-0031558 A1 | 14/03/2002 |
| | | US 2003-0186933 A1 | 02/10/2003 |
| | | US 2005-0158408 A1 | 21/07/2005 |
| | | US 2006-0089331 A1 | 27/04/2006 |
| | | US 2006-0188530 A1 | 24/08/2006 |
| | | US 2007-0072828 A1 | 29/03/2007 |
| | | US 2008-0057133 A1 | 06/03/2008 |
| | | US 6251428 B1 | 26/06/2001 |
| | | US 7166299 B2 | 23/01/2007 |
| | | US 7303768 B2 | 04/12/2007 |
| | | US 7772220 B2 | 10/08/2010 |
| | | US 7932243 B2 | 26/04/2011 |
| | | WO 00-04875 A2 | 03/02/2000 |
| | | WO 00-04875 A3 | 03/05/2001 |
| | | WO 2006-044771 A2 | 27/04/2006 |
| | | WO 2006-044771 A3 | 29/03/2007 |
| US 2014-0323455 A1 | 30/10/2014 | US 9724357 B2 | 08/08/2017 |
| | | WO 2013-025840 A1 | 21/02/2013 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *British Journal of Ophthalmology,* 2006